# EUROPEAN PATENT SPECIFICATION

(11) **EP 2 964 782 B1**
(45) Date of publication and mention of the grant of the patent: **10.01.2018**
(21) Application number: 14708310.9
(22) Date of filing: 07.03.2014
(51) Int. Cl.: C12Q 1/68

(54) **MOLECULAR MARKERS IN BLADDER CANCER**
MOLEKULARE MARKER BEI BLASENKREBS
MARQUEURS MOLÉCULAIRES DANS LE CANCER DE LA VESSIE

(30) Priority: 08.03.2013 WO PCT/EP2013/054777
(43) Date of publication of application: 13.01.2016
(73) Proprietor: MDxHealth Research B.V., 6525 GA Nijmegen (NL)
(72) Inventor: SMIT, Franciscus Petrus, NL-6546 RN Nijmegen (NL); HESSELS, Daphne, NL-6546 RN Nijmegen (NL); SCHALKEN, Jacobus A., NL-6524 LH Nijmegen (NL)
(74) Representative: van Kooij, Adriaan
(86) International application number: PCT/EP2014/054501
(87) International publication number: WO 2014/135698

(56) References cited:
- EP-A1- 2 434 024
- WO-A1-2006/012522
- WO-A1-2012/178087
- US-A1- 2010 279 301
- US-A1- 2012 058 477
- STRAVOPODIS DIMITRIOS J; KARKOULIS PANAGIOTIS K; KONSTANTAKOU EUMORPHIA G; MELACHROINOU SOPHIA; LAMPIDONIS ANTONIS D; ANASTASIOU D: "Grade-dependent effects on cell cycle progression and apoptosis in response to doxorubicin in human bladder cancer cell lines.", INTERNATIONAL JOURNAL OF ONCOLOGY, vol. 34, no. 1, 1 January 2009 (2009-01-01), pages 137-160, XP055119799, GR ISSN: 1019-6439, DOI: 10.3892/ijo_00000137
- DIANA ABDUEVA ET AL: "Experimental Comparison and Evaluation of the Affymetrix Exon and U133Plus2 GeneChip Arrays", PLOS ONE, vol. 2, no. 9, 1 January 2007 (2007-01-01) , pages e913-e913, XP055119867, ISSN: 1932-6203, DOI: 10.1371/journal.pone.0000913

## Description

The present invention relates to methods for establishing the prediction of prognosis and disease outcome for a human individual suffering from bladder cancer.

Urinary bladder (or bladder) cancer is one of the most common cancers worldwide, with the highest incidence in industrialized countries. In the Western world, the chances of developing this type of cancer is 1 in 26, for women the chance is 1 in 90. Bladder cancer is the 4th most common cancer in men.

Two main histological types of bladder cancer are the urothelial cell carcinomas (UCC) and the squamous cell carcinomas (SCC). The UCCs are the most prevalent in Western and industrialized countries and are related to cigarette smoking and occupational exposure. The squamous cell carcinomas (SCC) are more frequently seen in some Middle Eastern and African countries where the schistosoma haematobium parasite is endemic.

In the Western world, 90% of the bladder tumours are UCCs, 3 to 5% are SCCs, and 1 to 2% are adenocarcinomas. Two third of the patients with UCC can be catogorized into non-muscle invasive bladder cancer (NMIBC) and one third in muscle invasive bladder cancer (MIBC).

In NMIBC, the disease is generally confined to the bladder mucosa (stage Ta, carcinoma in situ (CIS)) or bladder submucosa (stage T1). In MIBC, the patient has a tumour initially invading the detrusor muscle (stage T2), followed by the perivesical fat (stage T3) and the organs surrounding the bladder (stage T4). The management of these two types of UCC differs significantly. The management of NMIBC consists of transurethral resection of the bladder tumour (TURBT). However, after TURBT, 30% to 85% of patients develop recurrences. This high risk of recurrence makes bladder cancer one of the most prevalent human tumours.

Patients with NMIBC can be divided into 3 groups. 20% to 30% of patients have a relatively benign type of UCC, with a low recurrence rate. These low risk tumours do not exhibit progression. 40% to 50% of patients have so-called intermediate risk tumours. These patients often develop a superficial recurrence, but seldom progression. A small group of patients (20% to 30%) has a relatively aggressive superficial tumour at presentation and despite maximum treatment and 70% to 80% of these patients will have recurrent disease. 50% of these patients will develop muscle invasive disease associated with a poor prognosis. Therefore, there is a need to identify the patient group at risk for progression.

The primary treatment for MIBC is cystectomy. Despite this radical treatment, 50% of patients with primary MIBC develop metastases within 2 years after cystectomy and subsequently die of the disease. The 5-year tumour-specific survival of these patients is 55%. In comparison, patients with NMIBC have a 5-year tumour-specific survival of 88-90%. However, patients with MIBC who have a history of NMIBC, the 5-year tumour-specific survival drops to only 28%. These percentages emphasize the need for the identification of patients with a high risk of progression of their NMIBC.

The risk for progression and cancer related death is associated with tumour stage and grade. Currently, staging and grading of the tumour is used for making treatment decisions. Unfortunately, this procedure has led to overtreatment (*e.g*. cystectomy in patients who would have survived without this treatment) or undertreatment (*i*.*e*. patients with progressive disease dying after TURBT and who would have survived if they underwent cystectomy at an earlier stage). At present no reliable methods are available to accurately predict prognosis of individual patients with bladder cancer. The limited value of the established prognostic markers requires the analysis of new molecular parameters in predicting the prognosis and treatment of bladder cancer patients.

Bladder cancer is a genetic disorder driven by the progressive accumulation of multiple genetic and epigenetic changes. At the molecular level, these genetic changes result in uncontrolled cell proliferation, decreased cell death, invasion, and metastasis. The specific alterations in gene expression that occur as a result of interactions between various cellular pathways determine the biological behavior of the tumor, including growth, recurrence, progression, and metastasis, and may influence patient survival. To detect and monitor cancer and determine the likely prognosis, it is necessary to identify molecular markers of the disease that can be used in the clinic.

US 2010/279301 discloses compositions and methods for cancer diagnosis, research and therapy, including but not limited to, cancer markers. In particular, disclosed are genes and gene panels as diagnostic markers and clinical targets for bladder cancer. Methods, compositions, and kits for diagnosing, determining, and determining risk of tumor aggressiveness are also disclosed.

EP 2 434 024 discloses that the BTM or UBTM family can be highly and consistently accumulated in bladder tumor tissue and other tumor tissue, and/or can be accumulated in urine of patients, and thus are markers for bladder and other types of cancer. In certain embodiments disclosed, BTMs or UBTMs can accumulate in the urine, and detection of UBTM family members can be an effective diagnostic approach.

Considering the above, there is a need in the art for molecular markers capable of stablishing the prediction of prognosis and disease outcome for a human individual suffering from bladder cancer. A suitable molecular marker preferably fulfils the following criteria:
1) it must be reproducible (intra- and inter- institutional); and
2) it must have an impact on clinical management.

It is an object of the present invention, amongst other objects, to meet at least partially, if not completely, the above stated needs of the art.

According to the present invention, the above object, amongst other objects, is met by methods as outlined in the appended claims.

Specifically, the above object, amongst other objects, is met by an (*in vitro*) method classification of the tumours according to aggressiveness, prediction of prognosis and/or disease outcome for a human individual suffering from bladder cancer comprising:
a) determining the expression of one or more genes chosen from the group consisting of CCNB2 in a sample originating from said human individual; and
b) establishing up regulation of expression as compared to expression of said gene in a sample originating from said human individual not comprising tumour cells or tissue, or from an individual, or group of individuals, not suffering from bladder cancer; and
c) establishing the prediction of prognosis and disease outcome for a human individual suffering from bladder cancer based on the established up regulation of said gene.

It should be noted that the present method, when taken alone, does not suffice to diagnose an individual as suffering from bladder cancer. For this, a trained physician is required capable of taking into account factors not related to the present invention such as disease symptoms, history, pathology, general condition, age, sex, and/or other indicators. The present methods and molecular markers provide the trained physician with additional tools, or aids, to arrive at a reliable diagnosis.

According to the present invention, expression analysis comprises establishing an increased expression of a gene as compared to expression of this gene in non-bladder cancer tissue, i.e., under non-disease conditions.

Establishing an increased expression of CCNB2 as compared to expression of these genes under non-bladder cancer conditions, allows establishing the prediction of prognosis and disease outcome for an individual patient suffering from bladder cancer.

CCNB2: Cyclin B2 is a member of the cyclin protein family. Cyclins B1 and B2 are particularly critical for the maintenance of the mitotic state. Cyclin B2 has been found to be up-regulated in human tumors, such as colorectal cancer, lung cancer, pituitary cancer. Recently it was shown that circulating CCNB2 in serum was significantly higher in cancer patients than in normal controls. The CCNB2 mRNA level was correlated with cancer stage and metastases status of patients with lung cancer and digestive tract cancer.

According to the present invention, the method as described above is an *ex vivo* or *in vitro* method. Expression analysis of the indicated genes is performed on a sample derived, originating or obtained from an individual suspected of suffering from bladder cancer. Such sample is urine. Samples of originating from urine such as urine sediments are contemplated within the context of the present invention as are samples of, derived or originating from TURBT specimens.

According to another preferred embodiment of the present method, determining the expression comprises determining mRNA expression.

Expression analysis based on mRNA is generally known in the art and routinely practiced in diagnostic labs world-wide. For example, suitable techniques for mRNA analysis are Northern blot hybridisation and amplification based techniques such as PCR, and especially real time PCR, and NASBA.

According to a particularly preferred embodiment, expression analysis comprises high-throughput DNA array chip analysis not only allowing the simultaneous analysis of multiple samples but also an automatic processing of the data obtained.

Preferred combinations within the context of the present invention are CCNB2 in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896, such as in combination with CDC20 and, preferably further in combination with PDCD1LG2, more preferably further in combination with INHBA, i.e. the combination at least comprising CCNB2, CDC20, PDCD1LG2 and INHBA. The latter panel of four markers provides a prediction of 0.991 (95%CI: 0.977-1.000). Within the present group of combinations with CCNB2, the samples are urine or urine derived samples such as urine sediments.

Disclosed is establishing metastasis or no metastasis. Establishing whether the bladder tumour identified is capable to metastasize, is likely to metastasize, or has metastasized, is inherently a valuable tool for a trained physician to develop an individualised treatment protocol.

In case of metastasis, the survival rate of a patient is generally directly correlated with the point in time on which the metastasis is identified, detected or established. The earlier in time the treatment commences, the higher the survival rates. Additionally, if a tumour is not capable of metastasis, is not likely to metastasize, or has not metastasized, the patient needs not to be subjected to, or can be spared of, treatments severely affecting the quality of life.

Establishing the presence, or absence, of a tumour, according to another preferred embodiment, can further comprise establishing whether a NMIBC wil, or is likely to, progress into MIBC.

Disclosed is the use of expression analysis of one or more genes selected from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and transcript cluster 2526896 for establishing the presence, or absence, of a bladder tumour or establishing the prediction of prognosis and disease outcome for an individual patient suffering from bladder cancer.

The above use, for reasons indicated above, can be an *ex vivo* or *in vitro* use and, can involve the use of two or more, three or more, four or more , five or more, six or more, seven or more, eight or more, nine or more, ten or more, eleven or more, twelve or more, thirteen or more, fourteen or more, fifteen or more, sixteen or more, seventeen or more and eighteen of the markers for establishing the presence, or absence of a bladder tumour, and establishing the prediction of prognosis and disease outcome for an individual patient suffering from bladder cancer.

Disclosd combinations within the context of this use are CCNB2 in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896, such as in combination with CDC20 and, preferably further in combination with PDCD1LG2, more preferably further in combination with INHBA, i.e. the combination at least comprising CCNB2, CDC20, PDCD1LG2 and INHBA. The latter panel of four markers provides a prediction of 0.991 (95%CI: 0.977-1.000). Within the present group of combinations with CCNB2, the preferred samples are urine or urine derived samples such as urine sediments.

Also disclosed are combinations within the context of this use are FAP in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896 such as in combination with CDC20 and, preferably, further in combination with INHBA, more preferably further in combination with IGF2BP2, i.e. the combination at least comprising FAP, CDC20, INHBA and IGF2BP2. Within the present group of combinations with FAP, the preferred samples are tissue or tissue derived samples such as biopses. The Area Under the Curve (AUC) for the combination of IGF₂BP₂+FAP+CHI₃L₁+CDC₂₀ expression is 0.955 (95%CI: 0.929-0.980).

Considering the diagnostic and/or prognostic value of the present genes as biomarkers for bladder cancer, disclosed is a kit of parts for establishing the presence, or absence, of a bladder tumour and establishing the prediction of prognosis and disease outcome for an individual patient suffering from bladder cancer said kit of parts comprises:
- expression analysis means for determining the expression of one or more genes chosen from the group consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and transcript cluster 2526896;
- instructions for use.

Combinations included in these kits are CCNB2 in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896, such as in combination with CDC20 and, preferably further in combination with PDCD1LG2, more preferably further in combination with INHBA, i.e. the combination at least comprising CCNB2, CDC20, PDCD1LG2 and INHBA.

Other combinations included in these kits are FAP in combination with one or more selected from the consisting of ADAMTS12, ASPN, CDC20, COL10A1, CTHRC1, FAP, SFRP4, FOXM1, KRT6A, ANLN, CHI3L1, TPX2, CCNB2, IGF2BP2, INHBA, PDCD1LG2, transcript cluster 2526893, and/or transcript cluster 2526896 such as in combination with CDC20 and, preferably, further in combination with INHBA, more preferably further in combination with IGF2BP2, i.e. the combination at least comprising FAP, CDC20, INHBA and IGF2BP2.

Disclosed is a kit of parts comprising mRNA expression analysis means, preferably for PCR, rtPCR or NASBA.

Disclosed is a kit of parts comprising means for expression analysis of two or more, three or more, four or more, five or more, six or more, seven or more, eight or more, nine or more, ten or more, eleven or more, twelve or more, thirteen or more, fourteen or more, fifteen or more, sixteen or more, seventeen or more or eighteen of the present genes.

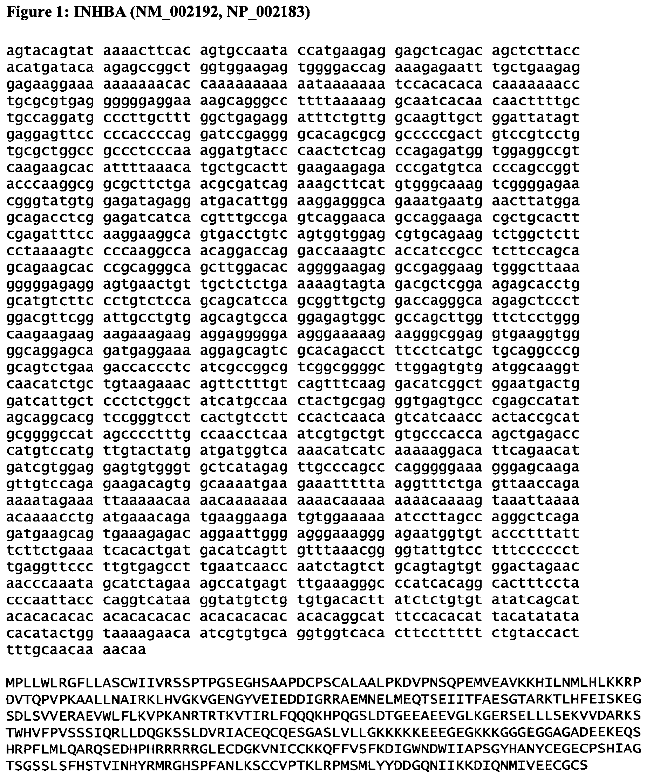

In the present description, reference is made to genes suitable as biomarkers for bladder cancer by referring to their arbitrarily assigned names. Although the skilled person is readily capable of identifying and using the present genes as biomarkers based on the indicated names, the appended figures 1 to 18 provide the cDNA and amino acid sequences of these genes, thereby readily allowing the skilled person to develop expression analysis assays based on analysis techniques commonly known in the art.

Such analysis techniques can, for example, be based on the genomic sequence of the gene or the provided cDNA or amino acid sequences. This sequence information can either be derived from the provided sequences, or can be readily obtained from public databases, for example by using the provided accession numbers.

The present invention will be further elucidated in the following examples of preferred embodiments of the invention. In the examples, reference is made to figures, wherein:
- **Figures 1-18**:: show the cDNA and amino acid sequences of the INHBA gene (NM_002192, NP_002183); the CTHRC1 gene (NM_138455, NP_612464); the CHI3L1 gene (NM_001276, NP_001267); the COL10A1 gene (NM_000493, NP_000484); the FAP gene (NM_004460, NP_004451); the sequence of transcript cluster 2526896 (no assigned mRNA and protein sequences); the ASPN gene (NM_017680, NP_060150); the sequence of transcript cluster 2526893 (no assigned mRNA and protein sequences); the ADAMTS12 gene (NM_030955, NP_112217); the IGF2BP2 gene (NM_006548, NP_006539); the PDCD1LG2 gene (NM_025239, NP_079515); the SFRP4 gene (NM_003014, NP_003005); the KRT6A gene (NM_005554, NP_005545); the TPX2 gene (NM_012112, NP_036244); the CCNB2 gene (NM_004701, NP_004692); the ANLN gene (NM_018685, NP_061155); the FOXM1 gene (NM_202002, NP_973731) and the CDC20 gene (NM_001255, NP_001246), respectively;
- **Figure 19**:: shows boxplots for the identified five best performing individual biomarkers that could distinguish NB1 from BCa (NMIBC+MIBC) in tissue;
- **Figure 20**:: shows boxplots for the identified five best performing individual biomarkers that could distinguish MIBC tissue from NMIBC tissue;
- **Figure 21**:: shows boxplots for the identified best performing individual biomarkers for the detection of BCa in urine and/or that could distinguish MIBC from NMIBC in urine;
- **Figure 22:**: shows receiver under Operation Curves (ROC) showing a combination of biomarkers for predicting the occurrence of BCa (NMIBC and MIBC) based on the expression of the markers in urine. The Area Under the Curve (AUC) for the combination of CCNB2+CDC20+PDCD1LG2+INHBA expression in urine is 0.991 (95%CI: 0.977-1.000)
- **Figure 23:**: shows Receiver under Operation Curves (ROC) showing a combination of biomarkers for predicting the occurrence of MIBC based on the expression of the markers in tissue. The Area Under the Curve (AUC) for the combination of IGF2BP2+FAP+CHI3L1+CDC20 expression in tissue is 0.955(95%CI: 0.929-0.980).

Below the present invention will be further illustrated by examples.

### EXAMPLES

### Example 1:

To identify markers for bladder cancer, the gene expression profile (GeneChip® Human Exon 1.0 ST arrays, Affymetrix) of samples from patients with and without bladder cancer were used. The expression analysis was performed according to standard protocols.

Briefly, tissue was obtained after radical cystectomy from patients with bladder cancer. The tissues were snap frozen and cryostat sections were hematoxylin-eosin (H.E.) stained for classification by a pathologist.

Malignant- and and non-malignant areas were dissected and total RNA was extracted with TRIpure® (Roche, Indianapolis, IN, CA, USA) following manufacturer's instructions. Total RNA was purified with the Qiagen RNeasy mini kit (Qiagen, Valencia, CA, USA). The integrity of the RNA was checked by electrophoresis using the Agilent 2100 Bioanalyzer.

From the purified total RNA, 1 µg was used for the GeneChip® Whole Transcript (WT) Sense Target Labeling Assay. (Affymetrix, Santa Clara, CA, USA). Using a random hexamer incorporating a T7 promoter, double-stranded cDNA was synthesized.

Then, cRNA was generated from the double-stranded cDNA template through an *in vitro* transcription reaction and purified using the Affymetrix sample clean-up module. Single-stranded cDNA was regenerated through a random-primed reverse transcription using a dNTP mix containing dUTP. The RNA was hydrolyzed with RNaseH and the cDNA was purified. Subsequently, the cDNA was fragmented by incubation with a mixture of UDG (uracil DNA glycosylase) and APE1 (apurinic/apyrimidinic endonuclease 1) restriction endonucleases and, finally, end-labeled via a terminal transferase reaction incorporating a biotinylated dideoxynucleotide. Of the fragmented, biotinylated cDNA, 5.5 µg was added to a hybridization mixture, loaded on a GeneChip® Human Exon 1.0 ST array and hybridized for 16 hours at 45 °C and 60 rpm.

Using the GeneChip® Human Exon 1.0 ST array, genes are indirectly measured by exon analysis which measurements can be combined into transcript clusters measurements. There are more than 300,000 transcript clusters on the array, of which 90,000 contain more than one exon. Of these 90,000 there are more than 17,000 high confidence (CORE) genes which are used in the default analysis. In total there are more than 5.5 million features per array.

Following hybridization, the array was washed and stained according to the Affymetrix protocol. The stained array was scanned at 532 nm using a GeneChip® Scanner 3000, generating CEL files for each array.

Exon-level and gene level expression values were derived from the CEL file probe-level hybridization intensities using Partek Genomics Suite 6.2, (Partek Incorporated, Saint Louis, MO, USA). Data analysis with this software was performed with the GeneChip® array core meta probe sets as well as the extended meta probe sets.

Differentially expressed genes between conditions, *e.g.* NMIBC *versus* MIBC and MIBC *versus* NBl, are calculated using Anova (ANalysis Of Variance), a T-test for more than two groups. The target identification is biased since clinically well-defined risk groups were analyzed. The markers are categorized based on their role in cancer biology. For the identification of markers the non-muscle invasive bladder cancer (NMIBC) group (N=48), the muscle invasive bladder cancer (MIBC) group (N=49), the bladder cancer metastasis (BC-Meta) group (N=5) and the normal bladder(NBl) group (N=12)were compared.

Based on the GeneChip® microarrays expression analysis data, the most differentially expressed genes between NBl and NMIBC/MIBC (diagnostic genes) and also the most differentially expressed genes between the NMIBC and MIBC (prognostic genes) were selected.
In total, a group of 46 genes of interest were selected which will be further elucidated in example 2 and listed in **Table 2**. Based on the selected 18 genes in example 2, the GeneChip® expression data for these genes are shown in **Table 1**.

**Table 1**: GeneChip® Microarray data showing the *expression characteristics of 18 targets characterizing bladder cancer tissue, based on the analysis of 12 well annotated NBl, 48 NMIBC, 49 MIBC and 5 BC-Meta tissue specimens.*

**Table 1A:**

| Gene | Gene | Gene | **up in MIBC vs NMIBC** | | up in MIBC vs NBl | |
|---|---|---|---|---|---|---|
| symbol | Name | assignment | Fold-Change | P-value | Fold-Change | P-value |
| INHBA | inhibin, beta A | NM_002192 | **10.1** | 3.3E-12 | 5.7 | 1.7E-8 |
| CTHRC1 | collagen triple helix repeat containing 1 | NM_138455 | **4.5** | 5.1E-22 | 2.4 | 7.9E-6 |
| CHI3L1 | chitinase 3-like 1 (cartilage glycoprotein-39) | NM_001276 | **13.5** | 1.5E-19 | 6.8 | 4.1E-7 |
| COL10A1 | collagen, type X, alpha 1 | NM_000493 | **3.7** | 1.0E-16 | 3.8 | 1.3E-9 |
| FAP | fibroblast activation protein, | NM_004460 | **6.3** | 2.5E-17 | 3.5 | 1.3E-5 |
| | alpha | | | | | |
| TC2526896* | Transcript cluster 2526896, N/A** | N/A** | **7.6** | 1.1E-15 | 7.8 | 5.6E-9 |
| ASPN | Asporin | NM_017680 | **7.6** | 1.1E-16 | 2.0 | 2.7E-2 |
| TC2526893* | Transcript cluster 2526893, N/A** | N/A** | **4.2** | 1.2E-12 | 4.2 | 1.8E-7 |
| ADAMTS12 | ADAM metallopeptidase with thrombospondin type 1 motif,12 | NM_016568 | **4.3** | 8.2E-21 | 3.8 | 1.9E-10 |
| IGF2BP2 | insulin-like growth factor 2 mRNA binding protein 2 | NM_006548 | **4.0** | 1.8E-12 | 2.7 | 2.5E-4 |
| PDCD1LG2 | programmed cell death 1 ligand 2 | NM_025239 | **5.6** | 1.2E-13 | 1.8 | 7.1E-2 |
| SFRP4 | secreted frizzled-related protein 4 | NM_003014 | **6.6** | 1.6E-12 | 2.9 | 3.5E-3 |
| KRT6A | keratin 6A | NM_005554 | **6.1** | 2.3E-07 | 4.6 | 2.6E-3 |

| | | | | | | |
|---|---|---|---|---|---|---|
| *data based on the GeneChip® extended meta probesets ** N/A = there are no assigned mRNA sequences for this transcript cluster. | | | | | | |

**Table 1B:**

| Gene | Gene | Gene | **up in MIBC vs NBl** | | up in MIBC vs NMIBC | |
|---|---|---|---|---|---|---|
| symbol | Name | assignment | Fold-Change | P-value | Fold-Change | P-value |
| TPX2 | TPX2, microtubule-associated, homolog (Xenopus laevis) | NM_012112 | **18.4** | 1.6E-19 | 2.6 | 3.9E-07 |
| CCNB2 | cyclin B2 | NM_004701 | **10.0** | 3.6E-19 | 1.6 | 5.6E-04 |
| ANLN | anilin, actin binding protein | NM_018685 | **16.3** | 1.8E-18 | 3.1 | 2.0E-09 |
| FOXM1 | forkhead box M1 | NM_202002 | **8.5** | 5.5E-18 | 2.4 | 2.2E-09 |
| CDC20 | cell devision cycle 20 homolog | NM_001255 | **29.0** | 6.4E-18 | 2.4 | 6.4E-05 |

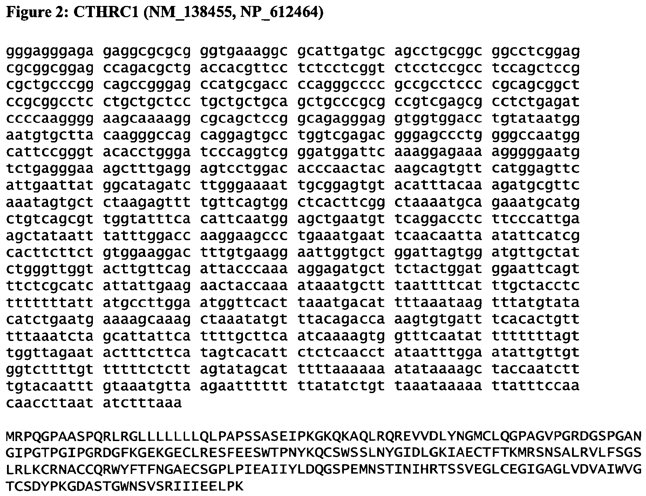

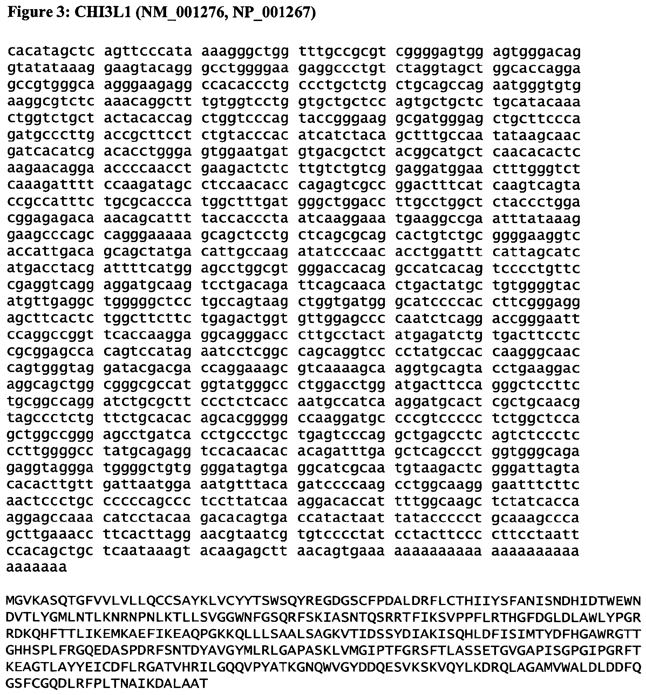

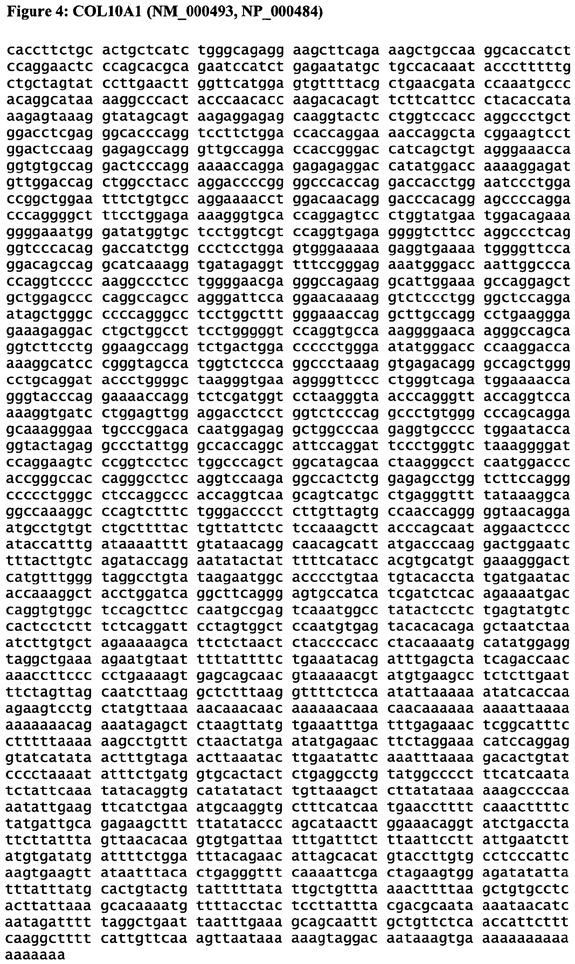

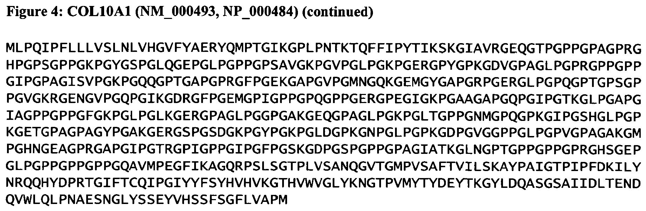

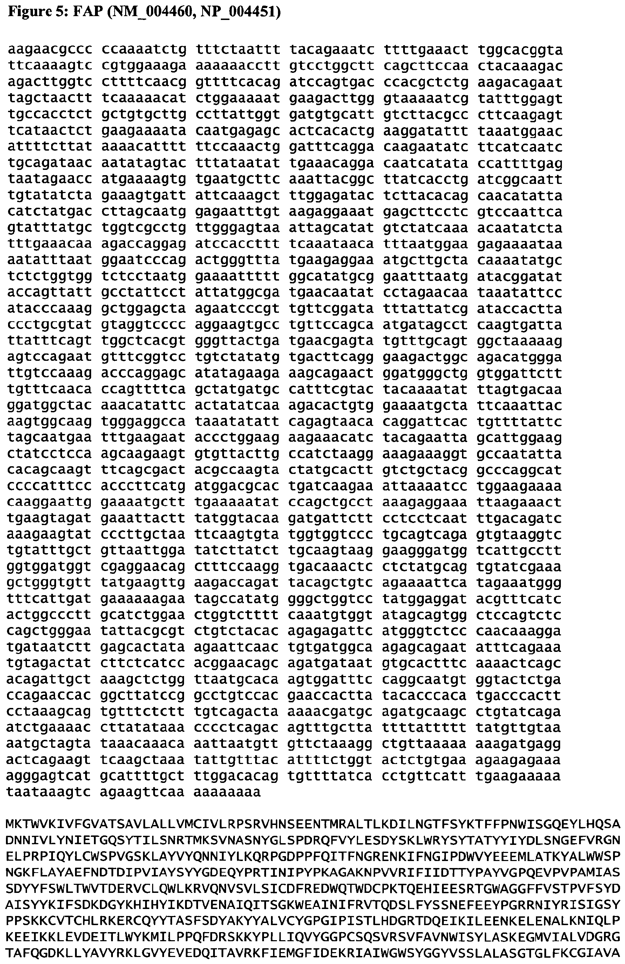

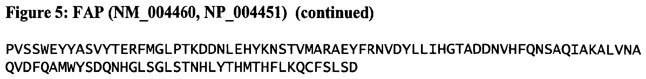

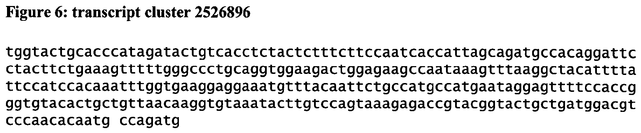

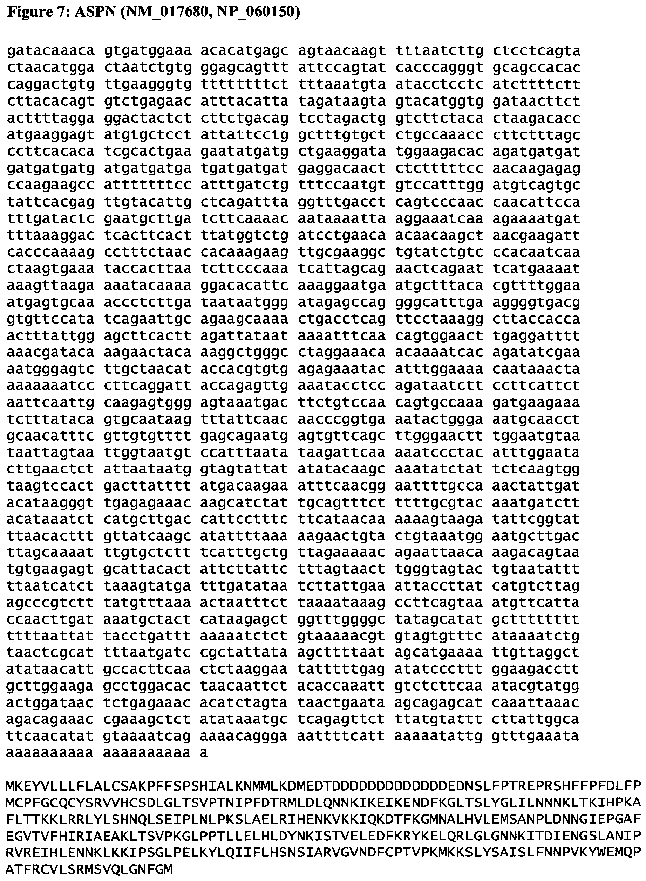

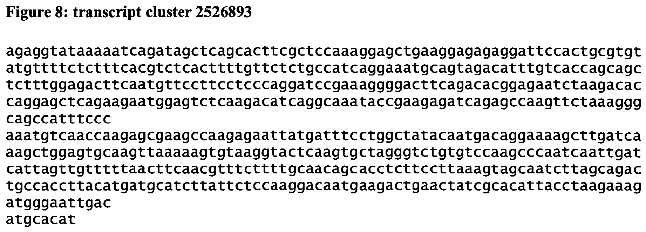

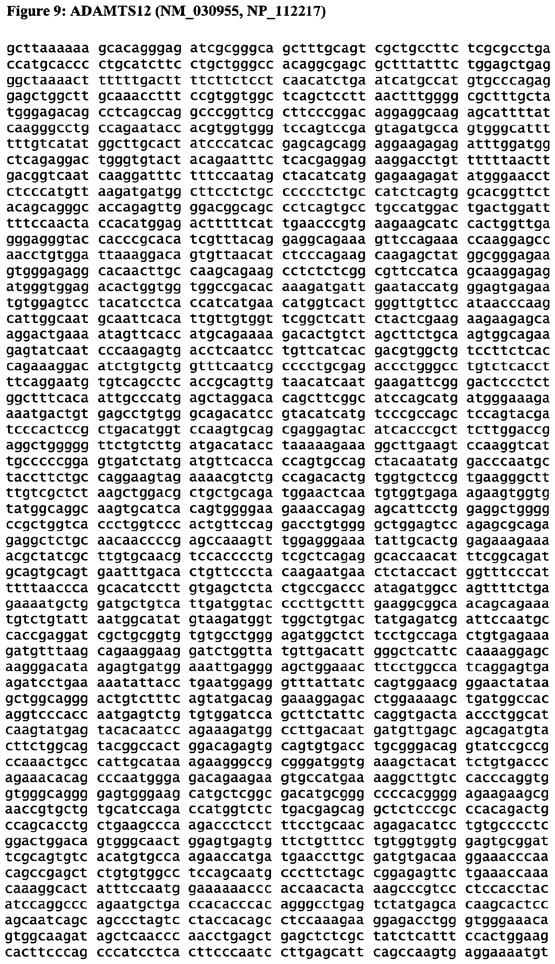

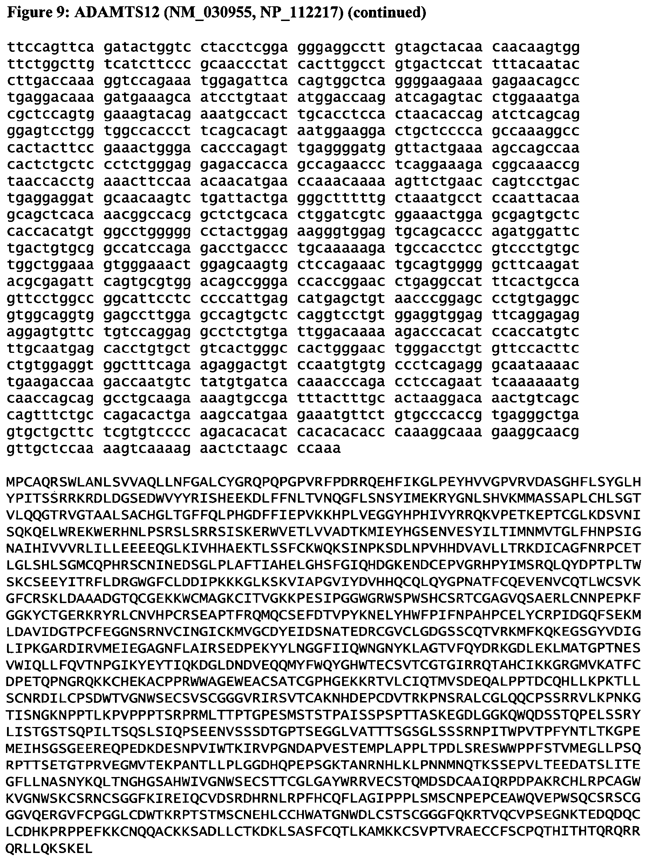

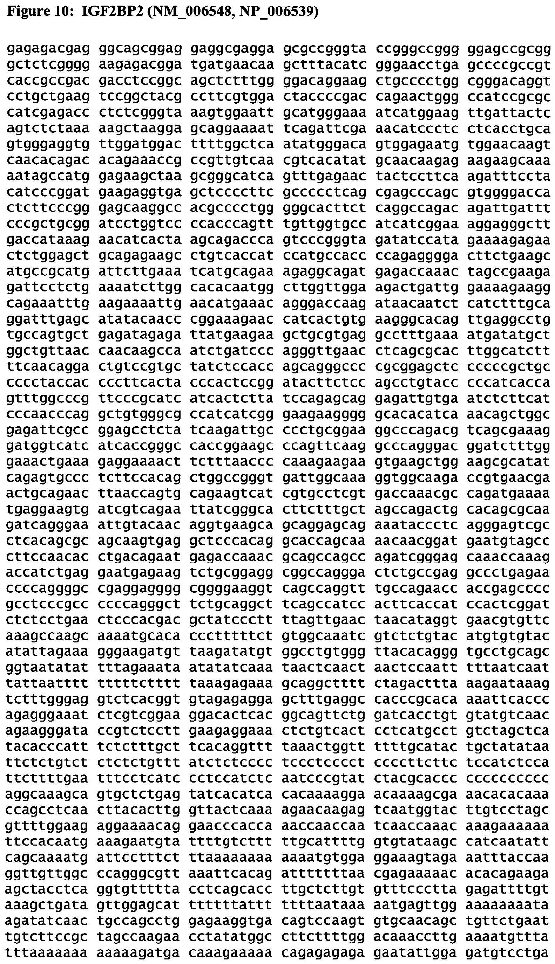

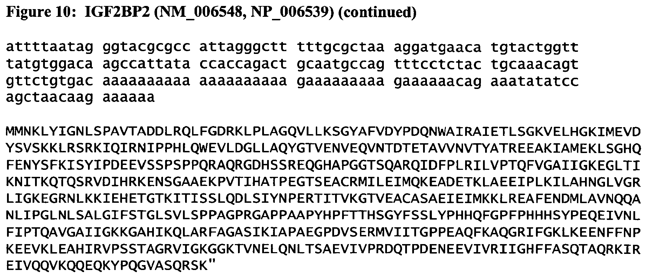

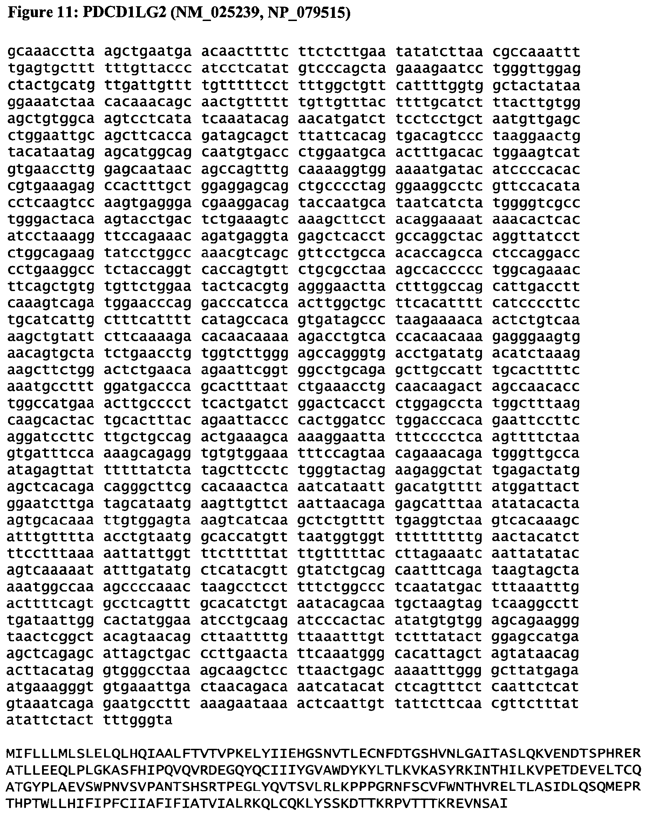

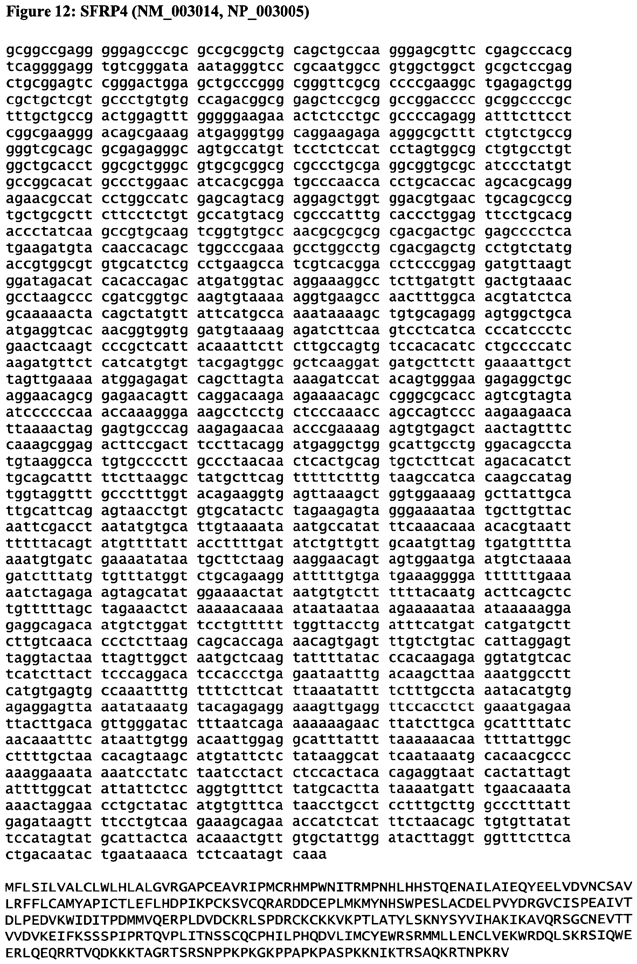

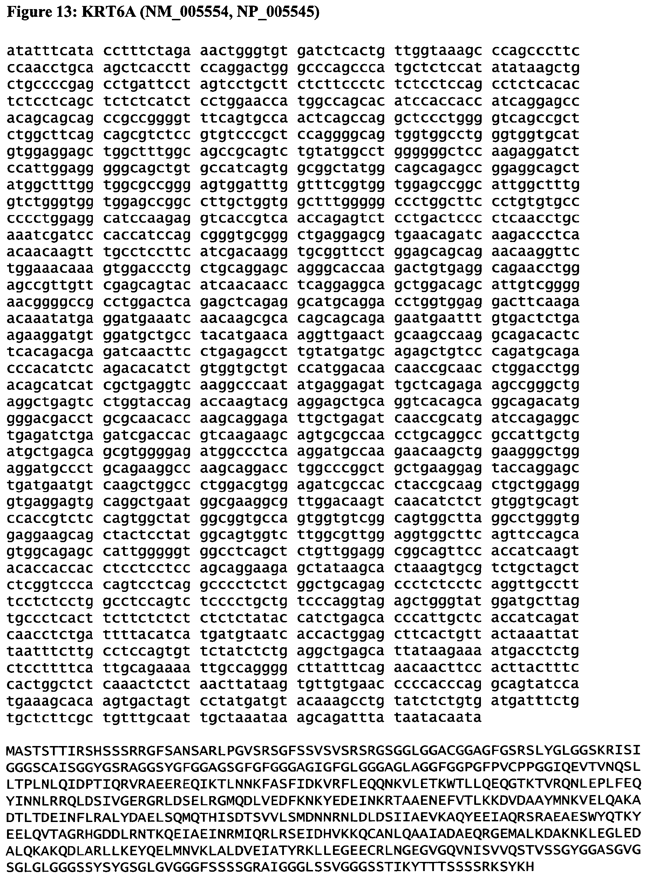

As can be clearly seen in **Table 1A** an up regulation of expression of INHBA (**Figures 1**), CTHRC1 (**Figures 2**), CHI3L1 (**Figures 3**), COL10A1 (**Figures 4**), FAP (**Figures 5**), transcript cluster 2526896 (**Figures 6**), ASPN (**Figures 7**), transcript cluster 2526893(Figures 8), ADAMTS12 (**Figures 9**), IGF2BP2 (**Figures 10**), PDCD1LG2 (**Figures 11**), SFRP4 (**Figure 12**), KRT6A (**Figure 13**) was associated with MIBC and as such has prognostic value. Eleven out of thirteen were identified using the core probe sets, two were identified using the extended probe sets and have no assigned mRNA sequence and gene symbol.

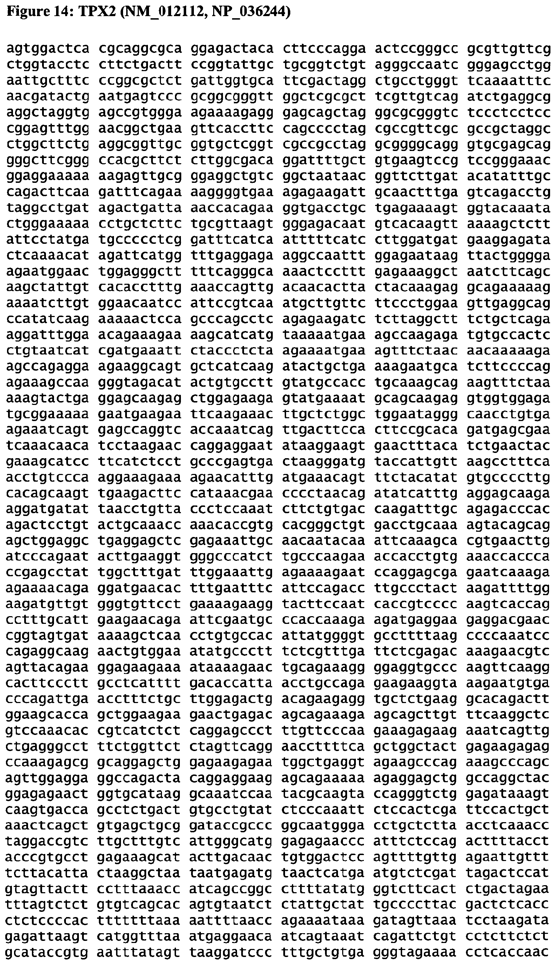

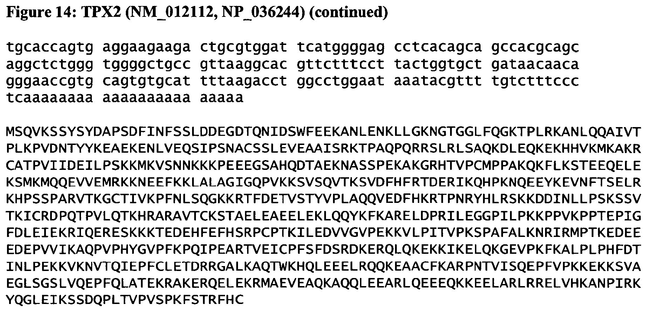

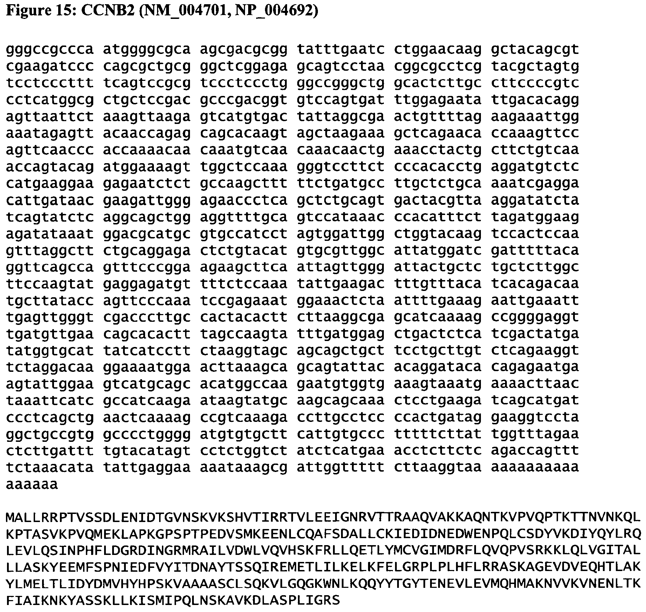

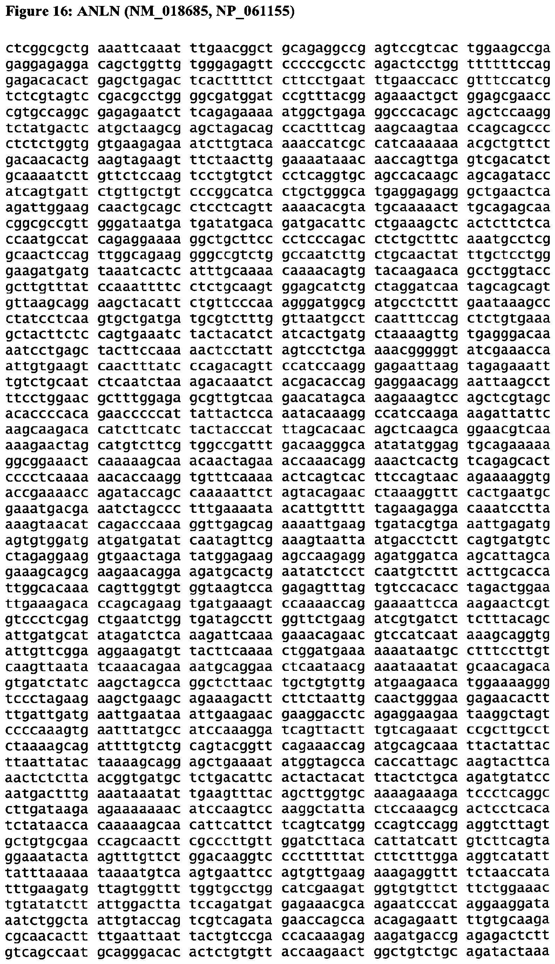

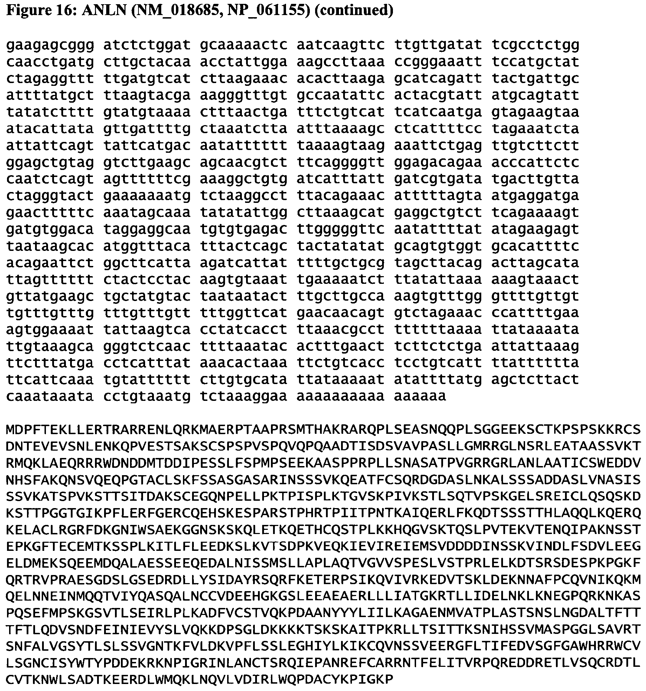

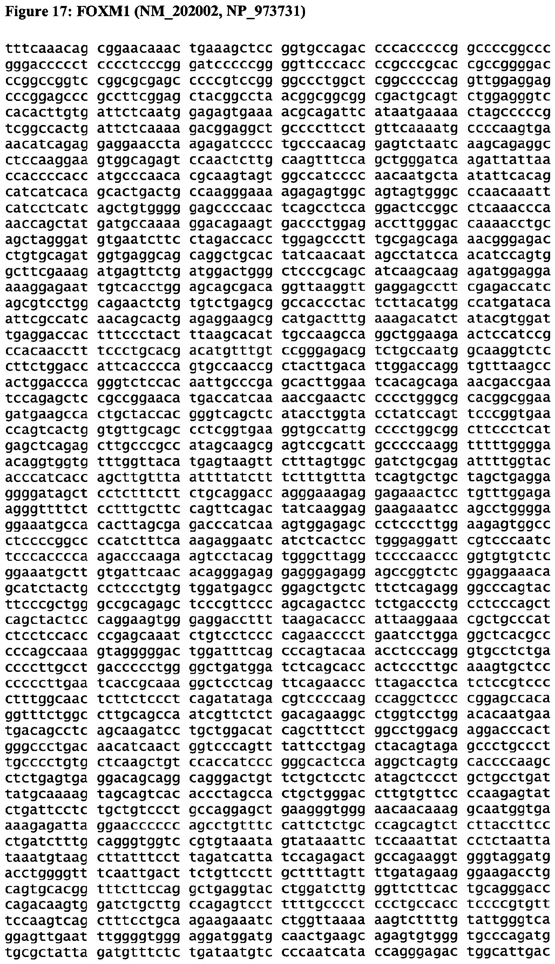

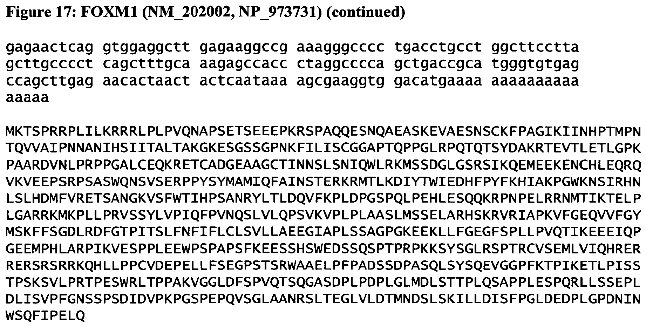

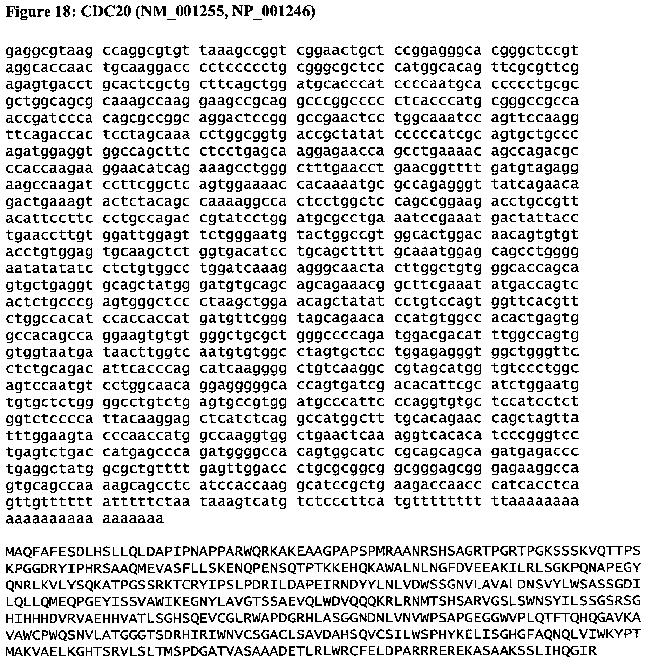

As can be clearly seen in **Table 1B** an up regulation of expression of TPX2 (**Figure 14**), CCNB2 (**Figure 15**), ANLN (**Figure 16**), FOXM1 (**Figure 17**) and CDC20 (**Figure 18**) was associated with the presence bladder cancer and as such has diagnostic value.

### Example 2

Using the gene expression profile (GeneChip® Human Exon 1.0 ST Array, Affymetrix) on 114 tissue specimens of normal bladder(NBl), non-muscle invasive bladder cancer (NMIBC), muscle invasive bladder cancer (MIBC) and bladder cancer metastasis (BC-Meta) several genes were found to be differentially expressed. The expression levels of 46 of these differentially expressed genes, together with the expression level of a housekeeping gene (GAPDH) and reference gene (TBP) were validated using the TaqMan® Low Density arrays (TLDA, Applied Biosystems). In **Table 2** an overview of the validated genes is shown.

**Table 2: Gene expression assays used for TLDA analysis**

| Gene symbol | Accesion nr. | Assay number |
|---|---|---|
| LOXL2 | NM_002318 | Hs00158757_m1 |
| INHBA | NM_002192 | Hs01081598_m1 |
| ADAMTS12 | NM_030955 | Hs00229594_m1 |
| CTHRC1 | NM_138455 | Hs00298917_m1 |
| SULF1 | NM_001128205 | Hs00290918_m1 |
| CHI3L1 | NM_001276 | Hs00609691_m1 |
| MMP11 | NM_005940 | Hs00968295_m1 |
| OLFML2B | NM_015441 | Hs00295836_m1 |
| CD109 | NM_133493 | Hs00370347_m1 |
| COL10A1 | NM_000493 | Hs00166657_m1 |
| NID2 | NM_007361 | Hs00201233_m1 |
| LOX | NM_002317 | Hs00942480_m1 |
| ADAMTS2 | NM_014244 | Hs01029111_m1 |
| FAP | NM_004460 | Hs00990806_m1 |
| GREM1 | NM_013372 | Hs01879841_m1 |
| WISP1 | NM_003882 | Hs00365573_m1 |
| ITGA11 | NM_001004439 | Hs00201927_m1 |
| ASPN | NM_017680 | Hs00214395_m1 |
| NTM | NM_001144058 | Hs00275411_m1 |
| PURR11 | NM_018304 | Hs00383634_m1 |
| BMP8A | NM_181809 | Hs00257330_s1 |
| SLC12A8 | NM_024628 | Hs00226405_m1 |
| SFRP4 | NM_003014 | Hs00180066_m1 |
| KRT6A | NM_005554 | Hs01699178_g1 |
| PDCD1LG2 | NM_025239 | Hs00228839_m1 |
| BCAT1 | NM_001178094 | Hs00398962_m1 |
| IGF2BP2 | NM_006548 | Hs01118009_m1 |
| TPX2 | NM_012112 | Hs00201616_m1 |
| CCNB2 | NM_004701 | Hs00270424_m1 |
| PLK1 | NM_005030 | Hs00153444_m1 |
| ANLN | NM_018685 | Hs01122612_m1 |
| AURKA | NM_198433 | Hs01582072_m1 |
| FOXM1 | NM_202002 | Hs01073586_m1 |
| CDC20 | NM_001255 | Hs00426680_mH |
| ECT2 | NM_018098 | Hs00216455_m1 |
| PLXNA1 | NM_032242 | Hs00413698_m1 |
| BUB1 | NM_004336 | Hs01557701_m1 |
| CKAP2 | NM_018204 | Hs00217068_m1 |
| TOP2A | NM_001067 | Hs00172214_m1 |
| TTK | NM_003318 | Hs01009870_m1 |
| CYB561D1 | NM_001134404 | Hs00699482_m1 |
| HMGB3 | NM_005342 | Hs00801334_s1 |
| SKP2 | NM_005983 | Hs01021864_m1 |
| FAPP6 | NM_001130958 | Hs01031183_m1 |
| FAM107A | NM_007177 | Hs00200376_m1 |
| NTRK3 | NM_001007156 | Hs00176797_m1 |
| TBP | NM_003194 | Hs00427620_m1 |
| GAPDH | NM_002046 | Hs99999905_m1 |

The validation with TLDA analysis was performed with 66 bladder tissue samples. Among these, 64 samples were newly selected and isolated, 2 normal bladder samples had been used before in the identification step with the GeneChip® Human Exon 1.0 ST Array.

Bladder cancer specimens in the following categories were used: Normal bladder (NBl, n=7), non-muscle invasive bladder cancer (NMIBC, n=29), muscle invasive bladder cancer (MIBC, n=27) and bladder cancer metastasis (BC-Meta, n=3).

To determine whether the identified biomarkers for bladder cancer could be used in a kit for specific detection in urine, 16 urinary sediments from patients suffering from bladder cancer (8 NMIBC and 8 MIBC) were included in the TLDA analysis and validation.

All tissue samples were snap frozen and cryostat sections were stained with hematoxylin and eosin (H.E.). These H.E.-stained sections were classified by a pathologist. Tumor areas were dissected. RNA was extracted from 10 µm thick serial sections that were collected from each tissue specimen at several levels. Tissue was evaluated by HE-staining of sections at each level and verified microscopically. Total RNA was extracted with TRIpure® (Roche, Indianapolis, IN, CA, USA) according to the manufacturer's instructions. Total RNA was purified using the RNeasy mini kit (Qiagen, Valencia, CA, USA).

The 16 urine samples of patients with bladder cancer were immediately cooled to 4°C and were processed within 48h after collection to guarantee good sample quality. The urine, EDTA stabilized, was centrifuged at 4°C and 1.800xg for 10 minutes. The obtained urinary sediment were washed twice with icecold buffered sodium chloride solution. On centrifugation at 4°C and 1.000xg for 10 minutes, the sediments were snap frozen in liquid nitrogen and stored at - 70°C. RNA was extracted from the urinary sediments using a modified TriPure reagent protocol. After the chloroform extraction, GlycoBlue was added to the aquous phase to precipitate the RNA using isopropanol. Total RNA from the sediments was used to generate amplified sense-strand cDNA using the Whole Transcriptome Expression kit according to the manufacturers protocol.

RNA quantity and quality were assessed on a NanoDrop 1000 spectrophotometer (NanoDrop Technologies, Wilmington, DE, USA) and on an Agilent 2100 Bioanalyzer (Agilent Technologies Inc., Santa Clara, CA, USA).

Two µg total RNA was eliminated from genomic DNA and reverse transcribed using the Quantitect® Reverse Transcription Kit Qiagen gMBH, Hilden, D) according to the manufacturer's instructions. Gene expression levels were measured using the TaqMan® Low Density Arrays (TLDA; Applied Biosystems).

A list of assays used in this study is given in **Table 2**. Of the individual cDNAs, 3 µl is added to 50 µl Taqman® Universal Probe Master Mix (Applied Biosystems)and 47 µl milliQ. One hundred µl of each sample was loaded into 1 sample reservoir of a TaqMan® Array (384-Well Micro Fluidic Card) (Applied Biosystems). The TaqMan® Array was centrifuged twice for 1 minute at 280g and sealed to prevent well-to-well contamination. The cards were placed in the micro-fluid card sample block of an 7900 HT Fast Real-Time PCR System (Applied Biosystems). The thermal cycle conditions were: 2 minutes 50°C, 10 minutes at 94.5°C, followed by 40 cycles for 30 seconds at 97°C and 1 minute at 59.7°C.

Raw data were recorded with the Sequence detection System (SDS) software of the instruments. Micro Fluidic Cards were analyzed with RQ documents and the RQ Manager Software for automated data analysis. Delta cycle threshold (Ct) values were determined as the difference between the Ct of each test gene and the Ct of glyceraldehyde 3-phosphate dehydrogenase (GAPDH) (endogenous control gene).

Furthermore, gene expression values were calculated based on the comparative threshold cycle (Ct) method, in which a normal bladder RNA sample was designated as a calibrator to which the other samples were compared.

For the validation of the differentially expressed genes found by the GeneChip® Human Exon 1.0 ST array, 66 bladder tissue specimens and 16 urinary sediments from bladder cancer patients were used in Taqman Low Density Arrays (TLDAs). In these TLDAs, expression levels were determined for the 48 genes of interest. The bladder tissue specimens were put in order from normal bladder, bladder cancer with low to high T-stage and finally bladder cancer metastasis.

Both GeneChip® Human Exon 1.0 ST array and TLDA data were analyzed using scatter- and box plots.

After analysis of the data a list of genes, shown in **Table 3**, was derived the expression of which is indicative for establishing the presence, or absence, of bladder tumour in a human individual suspected of suffering from bladder cancer comprising and, accordingly, indicative for bladder cancer and prognosis thereof.

**Table 3: List of genes identified**

| **Gene Symbol** | **Gene description** | **Figure** |
|---|---|---|
| INHBA | inhibin, beta A | 1 |
| CTHRC1 | collagen triple helix repeat containing 1 | 2 |
| CHI3L1 | chitinase 3-like 1 (cartilage glycoprotein-39) | 3 |
| COL10A1 | collagen, type X, alpha 1 | 4 |
| FAP | fibroblast activation protein, alpha | 5 |
| TC2526896* | transcript cluster 2526896, N/A** | 6 |
| ASPN | Aspirin | 7 |
| TC2526893* | transcript cluster 2526893, N/A** | 8 |
| ADAMTS12 | ADAM metallopeptidase with thrombospondin type 1 motif, 12 | 9 |
| IGF2BP2 | insulin-like growth factor 2 mRNA binding protein 2 | 10 |
| PDCD1LG2 | programmed cell death 1 ligand 2 | 11 |
| SFRP4 | secreted frizzled-related protein 4 | 12 |
| KRT6A | keratin 6A | 13 |
| TPX2 | TPX2, microtubule-associated, homolog (Xenopus laevis) | 14 |
| CCNB2 | cyclin B2 | 15 |
| ANLN | anilin, actin binding protein | 16 |
| FOXM1 | forkhead box M1 | 17 |
| CDC20 | cell devision cycle 20 homolog | 18 |

| | | |
|---|---|---|
| *data based on the GeneChip® extended meta probesets ** N/A = there are no assigned mRNA sequences for this transcript cluster. | | |

Below detailed GeneChip® Human Exon 1.0 ST array data and TLDA validation data is presented for the 16 genes and only GeneChip® array data for the two transcript clusters, based on the groups normal bladder(NBl), non-muscle invasive bladder cancer (NMIBC), muscle invasive bladder cancer (MIBC) and bladder cancer metastasis (BC-Meta). For the identification of markers the non-muscle invasive bladder cancer (NMIBC) group, the muscle invasive bladder cancer (MIBC) group, the bladder cancer metastasis (BC-Meta) group and the normal bladder(NBl) group were compared.

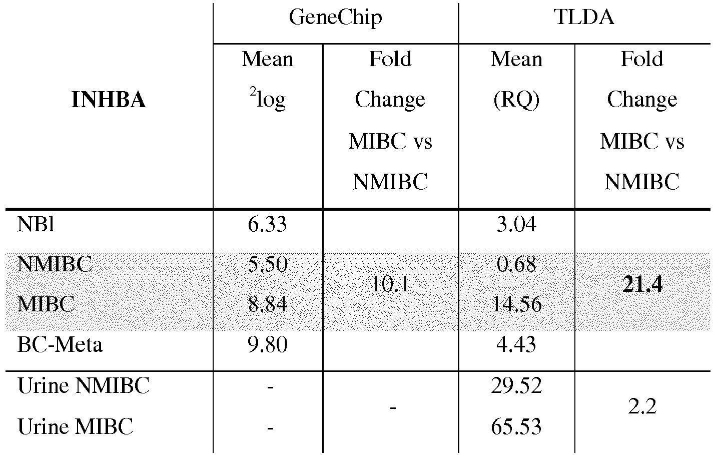

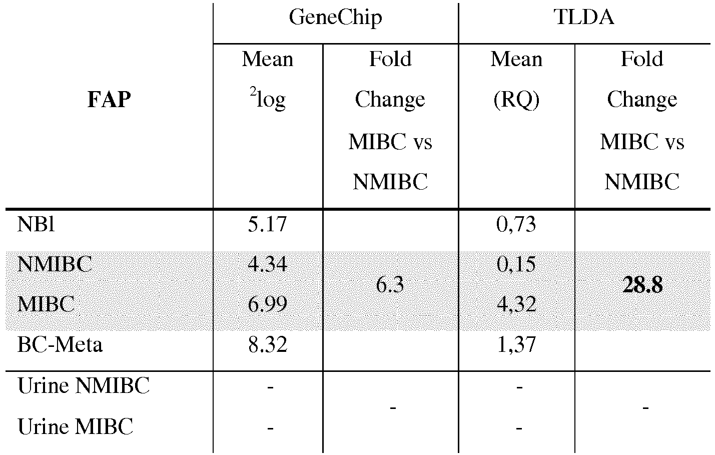

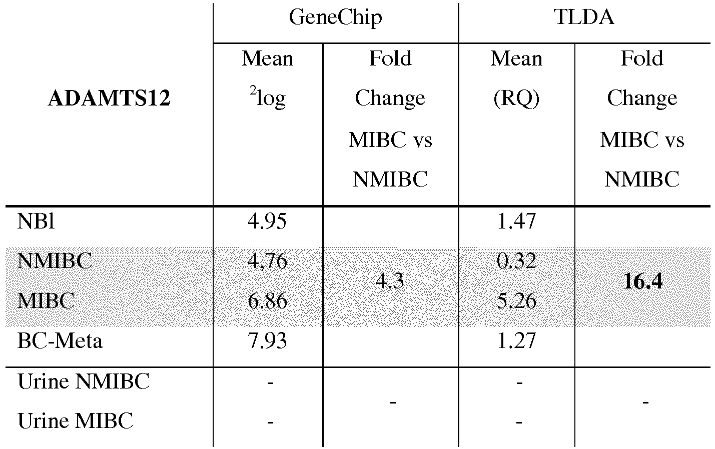

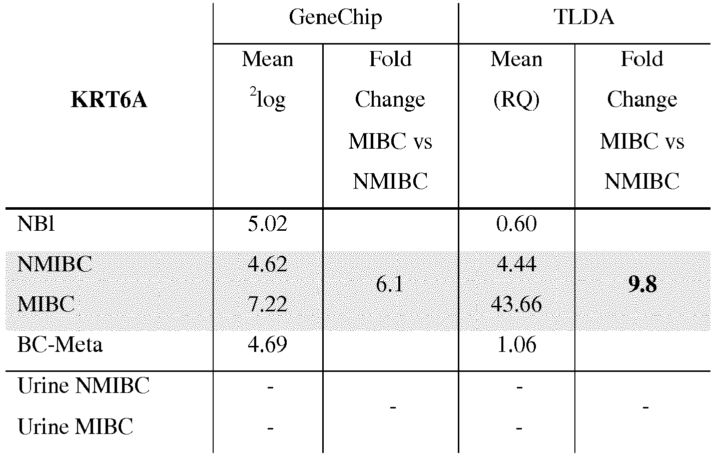

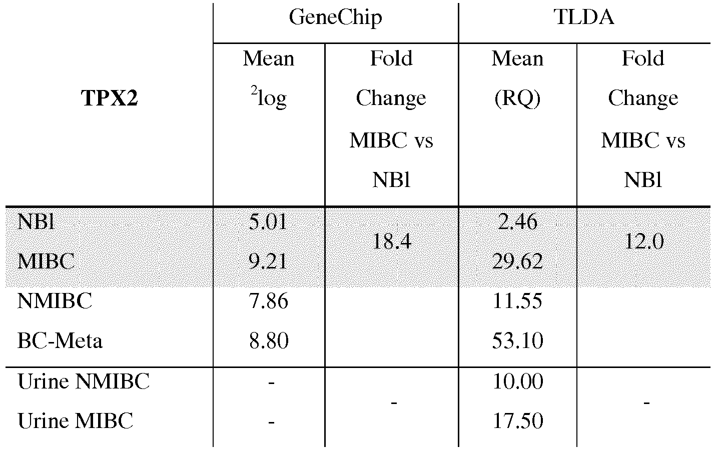

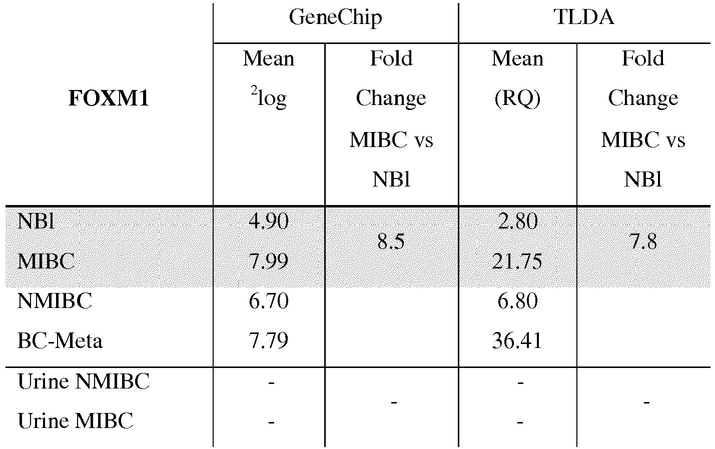

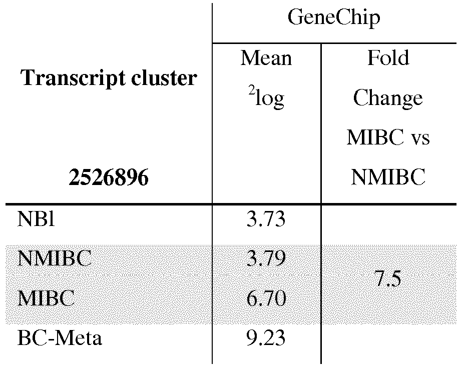

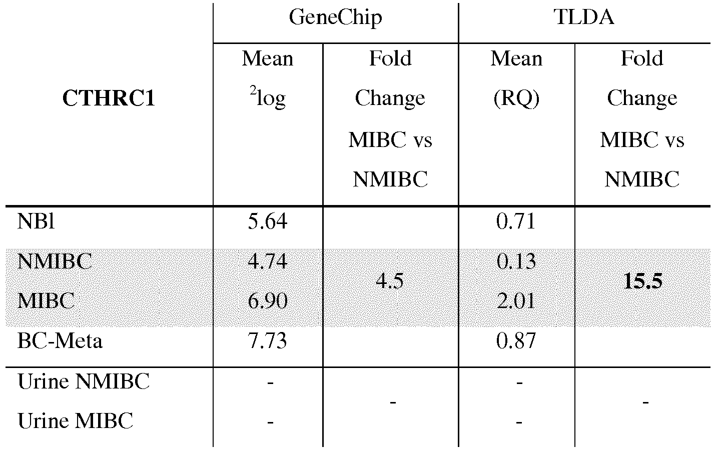

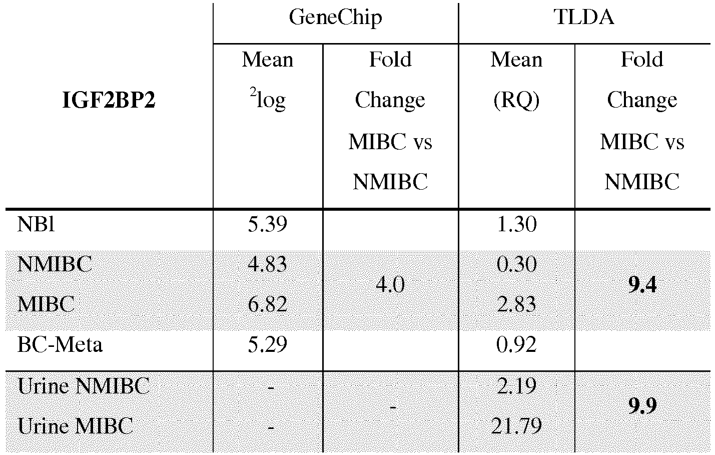

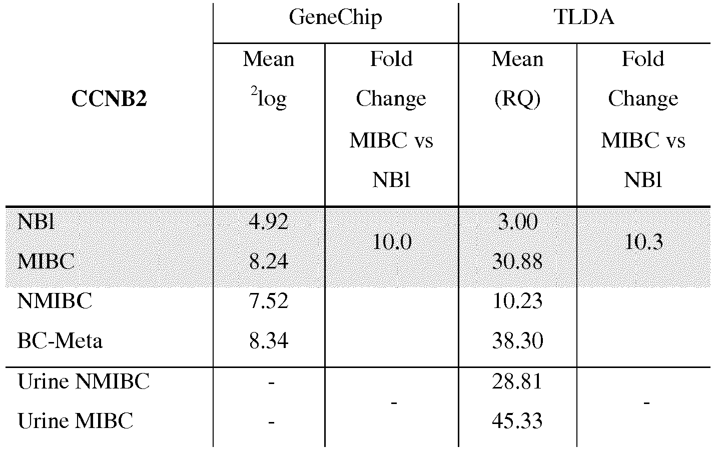

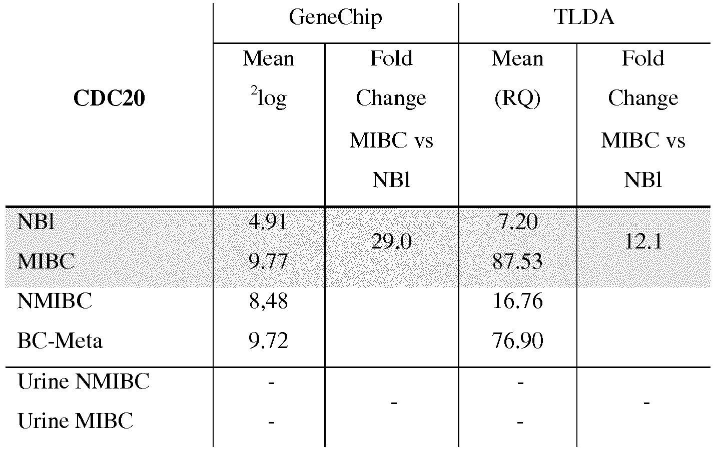

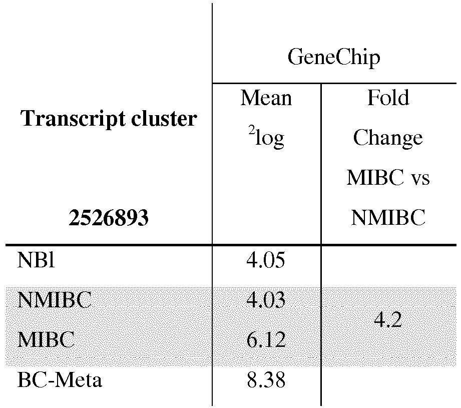

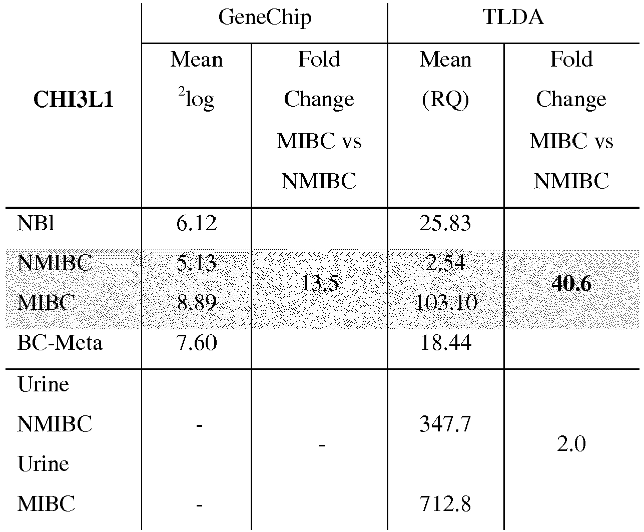

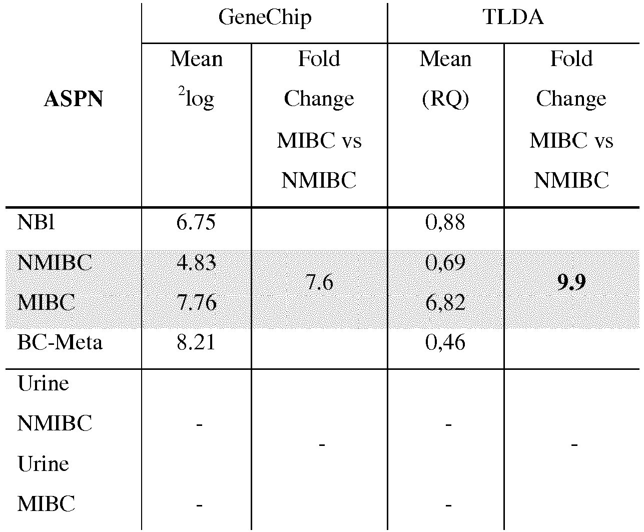

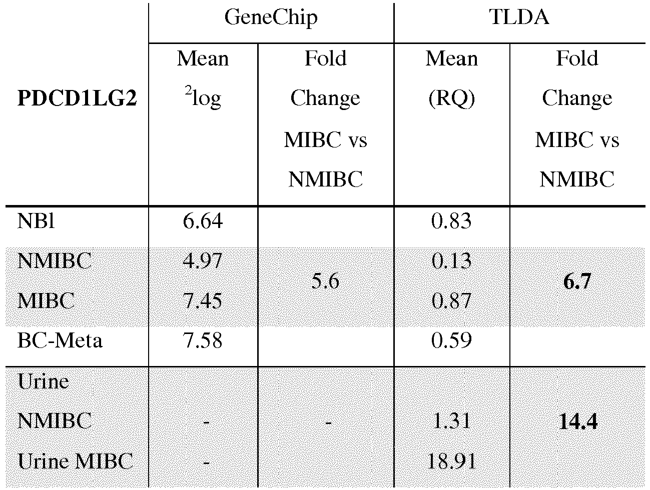

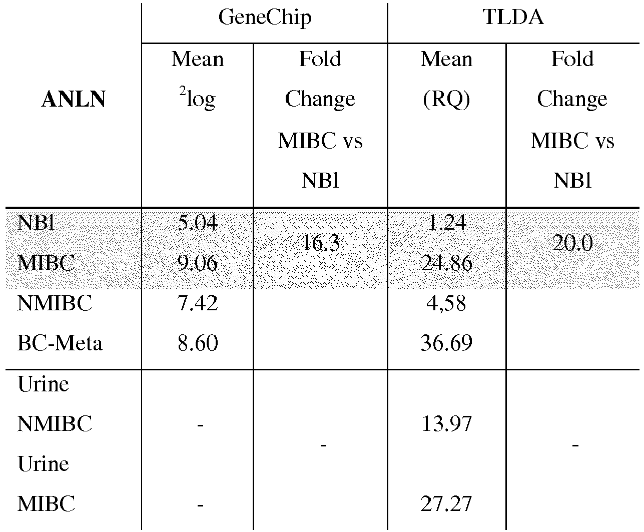

### Example 3:

The identified genes mentioned in example 2 and listed in **Table 3** were used for further validation and selection in a larger cohort of patient samples. For 17 of the 18 identified genes and for the control gene TBP used for normalization, fluorescence based real-time qPCR assays were designed and established according the MIQE guidelines. The performance of transcript clusters 2526896 and 2526893 were very similar. Therefore, no qPCR assay was established for transcript cluster 2526893. PCR products were cloned in either the pCR2.1-TOPO cloning vector (Invitrogen). Calibration curves with a wide linear dynamic range (10 - 1,000,000 copies) were generated using serial dilutions of the plasmids. The amplification efficiency of the primer pair was determined using the calibration curve and was >1.85. Control samples with known template concentrations were used as a reference. Two µl of each cDNA sample were amplified in a 20 µl PCR reaction containing optimized amounts of forward primer and reverse primer, 2 pmol of hydrolysis probe and 1x Probes Master mix (Roche, Cat No. 04902343001). The following amplification conditions were used: 95°C for 10 minutes followed by 50 cycles at 95°C for 10 seconds, 60°C for 30 seconds and a final cooling step at 40°C for 55 seconds (LightCycler LC480, Roche). The crossing point (Cp) values were determined using the Lightcycler 480 SW 1.5 software (Roche). The Cp values of the samples were converted to concentrations by interpolation in the generated calibration curve. The assay performance of the real-time PCR experiments was evaluated during in-study validation. The reference control samples had an inter- and intra-assay variation < 30%.

Total RNA was extracted from bladder tissue and urinary sediments and used for reverse transcription to generate cDNA. In total 211 bladder tissue specimen and 100 urinary sediments were used. The group of 206 bladder tissue specimen consisted of 10 normal bladders, 124 NMIBC, 72 MIBC. The group of 100 urinary sediments consisted of urinary sediments from 15 healthy controls (defined as normal), and from 65 patients with NMIBC, and 18 patients with MIBC.

Statistical analyses were performed with SPSS® version 20.0. All data were log-transformed prior to statistical analysis as a transformation to a normal distribution. Two-tailed P values of 0.05 or less were considered to indicate statistical significance. The nonparametric Mann Whitney test (for continuous variables) was used to test if biomarker levels were significantly correlated with the presence of BCa and/or BCa prognosis (muscle invasiveness, metastasis).

The assay results for the 17 selected biomarkers are shown in **Tables 4-7**.

**Table 4: Absolute and relative expression of the 17 biomarkers in NBl and BCa tissue**

| | copy numbers | | | copy numbers | | | relative expression¹ | | | |
|---|---|---|---|---|---|---|---|---|---|---|
| | NBl N=10 | | | BCa (NMIBC+MIBC) N=196 | | | NBl N=10 | BCa N=196 | Fold Change | P-value |
| Biomarker | Mean | Median | Range | Mean | Median | Range | Mean | Mean | | MW² |
| CTHRC1 | 1041 | 1263 | 1-1810 | 3129 | 835 | 1-42200 | 917.8 | 1891.7 | 2.1 | 0.29 |
| IGF2BP2 | 203 | 130 | 69-651 | 544 | 85 | 0-5750 | 182.1 | 248.8 | 1.4 | 0.74 |
| ADAMTS12 | 91 | 74 | 24-259 | 472 | 103 | 1-4180 | 75.5 | 275.8 | 3.7 | 0.31 |
| INHBA | 411 | 437 | 30-869 | 3472 | 386 | 1-61500 | 400.5 | 1895.9 | 4.7 | 0.38 |
| SFRP4 | 231 | 92 | 55-896 | 1365 | 26 | 1-37300 | 201.7 | 911.5 | 4.5 | 0.18 |
| FAP | 568 | 403 | 146-1440 | 1239 | 270 | 1-12500 | 469.2 | 738.0 | 1.6 | 0.52 |
| CHI3L1 | 81 | 52 | 1-339 | 1468 | 158 | 1-21700 | 104.5 | 883.2 | 8.5 | 0.45 |
| COL10A1 | 275 | 205 | 58-585 | 2991 | 984 | 1-88200 | 225.6 | 1881.5 | 8.3 | 0.56 |
| ASPN | 548 | 205 | 23-3500 | 608 | 210 | 1-15700 | 397.8 | 372.0 | -1.1 | 0.8 |
| ANLN | 92 | 35 | 1-412 | 1297 | 772 | 17-8000 | 56.9 | 521.8 | 9.2 | <0.05 |
| TPX2 | 73 | 28 | 1-325 | 1768 | 964 | 1-13000 | 45.8 | 715.0 | 15.6 | <0.05 |
| FOXM1 | 57 | 16 | 1-251 | 806 | 397 | 1-5650 | 32.9 | 271.3 | 8.2 | <0.05 |
| CCNB2 | 156 | 47 | 1-669 | 2160 | 1485 | 1-11100 | 93.8 | 811.1 | 8.6 | <0.05 |
| CDC20 | 185 | 54 | 1-795 | 2727 | 1650 | 1-22000 | 122.0 | 1057.5 | 8.7 | <0.05 |
| KRT6A | 1983 | 26 | 1-19500 | 32220 | 132 | 1-1470000 | 1386.7 | 12483.6 | 9.0 | 0.48 |
| PDCD1LG2 | 288 | 223 | 119-536 | 570 | 336 | 1-3440 | 262.1 | 298.0 | 1.1 | 0.14 |
| TC2526896 | 3 | 1 | 1-11 | 110 | 1 | 1-1920 | 1.9 | 63.8 | 33.6 | 0.67 |
| TBP | 1230 | 1058 | 492-2820 | 2890 | 2585 | 77-9320 | - | - | - | - |

Relative expression¹: ratio (copy numbers biomarker/copy number TBP)*1000 MW²: Mann-Whitney test

**Table 5: Absolute and relative expression of the 17 biomarkers in NMIBC and MIBC tissue**

| | copy numbers | | | copy numbers | | | ¹ relative expression | | | | |
|---|---|---|---|---|---|---|---|---|---|---|---|
| | NMIBC N=124 | | | MIBC N=72 | | | NMIBC N=124 | MIBC N=72 | Fold Change | P-value | P-value |
| Biomarker | Mean | Median | Range | Mean | Median | Range | Mean | Mean | | MW² | T-test |
| CTHRC1 | 898 | 585 | 24-5360 | 6972 | 4625 | 1-42200 | 364.6 | 4521.7 | 12.4 | <0.05 | 3.5E-20 |
| IGF2BP2 | 206 | 38 | 0-1950 | 1126 | 611 | 1-5750 | 74.2 | 549.4 | 7.4 | <0.05 | 1.0E-20 |
| ADAMTS12 | 124 | 65 | 1-1880 | 1070 | 515 | 1-4180 | 48.2 | 667.9 | 13.9 | <0.05 | 1.4E-19 |
| INHBA | 529 | 230 | 1-7440 | 8540 | 3775 | 29-61500 | 183.0 | 4846.0 | 26.5 | <0.05 | 1.IE-22 |
| SFRP4 | 76 | 1 | 1-2300 | 3567 | 509 | 1-37300 | 41.6 | 2367.6 | 56.9 | <0.05 | 3.3E-23 |
| FAP | 323 | 167 | 1-2510 | 2817 | 1915 | 39-12500 | 135.2 | 1776.3 | 13.1 | <0.05 | 9.6E-34 |
| CHI3L1 | 475 | 37 | 1-21700 | 3177 | 1420 | 15-14800 | 170.6 | 2110.5 | 12.4 | <0.05 | 2.6E-30 |
| COL10A1 | 1087 | 804 | 1-4670 | 6271 | 2040 | 1-88200 | 337.4 | 4540.6 | 13.5 | <0.05 | 2.3E-10 |
| ASPN | 223 | 134 | 1-1450 | 1271 | 551 | 1-15700 | 105.7 | 830.6 | 7.9 | <0.05 | 2.2E-18 |
| ANLN | 1001 | 582 | 17-6930 | 1806 | 1380 | 25-8000 | 318.5 | 871.9 | 2.7 | <0.05 | 2.6E-16 |
| TPX2 | 1358 | 579 | 1-8760 | 2474 | 1620 | 1-13000 | 430.5 | 1205.1 | 2.8 | <0.05 | 3.2E-10 |
| FOXM1 | 723 | 336 | 1-5650 | 948 | 515 | 1-3660 | 196.1 | 400.8 | 2.0 | <0.05 | 4.6E-07 |
| CCNB2 | 2075 | 1275 | 17-11100 | 2306 | 1575 | 1-7360 | 637.3 | 1110.4 | 1.7 | <0.05 | 6.2E-07 |
| CDC20 | 2062 | 1210 | 1-15300 | 3873 | 2445 | 1-22000 | 642.0 | 1773.0 | 2.8 | <0.05 | 3.7E-10 |
| KRT6A | 2257 | 71 | 1-181000 | 83822 | 2320 | 1-1470000 | 721.9 | 32739.8 | 45.4 | <0.05 | 1.3E-11 |
| PDCD1LG2 | 477 | 292 | 1-3200 | 728 | 413 | 1-3440 | 185.4 | 492.0 | 2.7 | <0.05 | 6.3E-10 |
| TC2526896 | 14 | 1 | 1-606 | 276 | 70 | 1-1920 | 4.4 | 166.7 | 37.9 | <0.05 | 1.IE-16 |
| TBP | 3354 | 3250 | 129-9320 | 2090 | 1675 | 77-9260 | - | - | - | - | - |

Relative expression¹: ratio (copy numbers biomarker/copy number TBP)*1000 MW²: Mann-Whitney test

**Table 6: Expression levels of the 17 biomarkers in normal bladder and BCa urine samples**

| | copy nu mbers | | | copy nu mbers | | | Fold Change | P-value |
|---|---|---|---|---|---|---|---|---|
| | NBl N=15 | | | BCa (NMIBC+MIBC) N=85 | | | | |
| Biomarker | Mean | Median | Range | Mean | Median | Range | | MW¹ |
| CTHRC1 | 1764 | 1030 | 58-5140 | 17901 | 10200 | 12-190000 | 10.1 | <0.005 |
| IGF2BP2 | 9994 | 6160 | 94-34600 | 135158 | 36700 | 1040-4000000 | 13.5 | <0.005 |
| ADAMTS12 | 1 | 1 | 1-1 | 110 | 1 | 1-1490 | 110.0 | 0.037 |
| INHBA | 10487 | 4220 | 1-70300 | 373104 | 89400 | 64-6070000 | 35.6 | <0.005 |
| SFRP4 | 24 | 1 | 1-210 | 181 | 50 | 1-1520 | 7.5 | 0.007 |
| FAP | 8 | 1 | 1-106 | 758 | 107 | 1-21000 | 94.8 | <0.005 |
| CHI3L1 | 53324 | 24600 | 2220-368000 | 754615 | 234000 | 2900-5380000 | 14.2 | <0.005 |
| COL10A1 | 1296 | 160 | 1-5630 | 7700 | 6130 | 1-43300 | 5.9 | <0.005 |
| ASPN | 122 | 1 | 1-632 | 75 | 1 | 1-1000 | 0.6 | 0.881 |
| ANLN | 2283 | 832 | 1-10700 | 37092 | 13600 | 25-454000 | 16.2 | <0.005 |
| TPX2 | 946 | 521 | 1-3120 | 28382 | 8860 | 1-310000 | 30.0 | <0.005 |
| FOXM1 | 231 | 209 | 1-649 | 12502 | 4000 | 16-165000 | 54.1 | <0.005 |
| CCNB2 | 1951 | 1290 | 12-10000 | 30857 | 16000 | 1120-240000 | 15.8 | <0.005 |
| CDC20 | 3490 | 2600 | 189-11100 | 40674 | 15800 | 280-61200 | 11.7 | <0.005 |
| KRT6A | 140105 | 70000 | 1230-762000 | 70419 | 27200 | 13-576000 | 0.5 | 0.362 |
| PDCD1LG2 | 1869 | 877 | 1-8650 | 52224 | 16300 | 1-679000 | 27.9 | <0.005 |
| TC2526896 | 2876 | 2840 | 557-6590 | 4356 | 3100 | 73-28600 | 1.5 | 0.382 |
| TBP | 29783 | 21100 | 1160-94400 | 199980 | 161000 | 6000-950000 | - | |

**Table 7: Expression levels of the 17 biomarkers in NMIBC and MIBC urine samples**

| | copy numbers | | | copy numbers | | | Fold Change | P-value |
|---|---|---|---|---|---|---|---|---|
| | NMIBC N=66 | | | MIBC N=19 | | | | |
| Biomarker | Mean | Median | Range | Mean | Median | Range | | MW¹ |
| CTHRC1 | 13991 | 9860 | 12-76100 | 31483 | 11400 | 1190-190000 | 2.3 | 0.143 |
| IGF2BP2 | 71976 | 31500 | 1040-1740000 | 365297 | 85250 | 2290-4000000 | 5.1 | 0.087 |
| ADAMTS12 | 71 | 1 | 1-790 | 253 | 1 | 1-1490 | 3.6 | 0.070 |
| INHBA | 288446 | 79500 | 64-3420000 | 667174 | 101000 | 13200-6070000 | 2.3 | 0.451 |
| SFRP4 | 153 | 13 | 1-919 | 276 | 123 | 1-1520 | 1.8 | 0.085 |
| FAP | 427 | 100 | 1-8390 | 1905 | 352 | 1-21000 | 4.5 | 0.023 |
| CHI3L1 | 689779 | 204000 | 22900-5050000 | 979837 | 394000 | 28300-5380000 | 1.4 | 0.117 |
| COL10A1 | 7596 | 6170 | 1-43300 | 8060 | 6130 | 409-28200 | 1.1 | 0.587 |
| ASPN | 54 | 1 | 1-436 | 151 | 1 | 1-1000 | 2.8 | 0.203 |
| ANLN | 31291 | 12450 | 25-454000 | 57243 | 21300 | 350-273000 | 1.8 | 0.083 |
| TPX2 | 16665 | 7180 | 1-188000 | 69081 | 14200 | 232-310000 | 4.1 | 0.017 |
| FOXM1 | 7218 | 3465 | 16-76800 | 30299 | 10600 | 101-156000 | 4.2 | 0.008 |
| CCNB2 | 22387 | 14900 | 1120-161000 | 60280 | 27300 | 1130-240000 | 2.7 | 0.014 |
| CDC20 | 22898 | 12700 | 396-184000 | 102422 | 23700 | 280-612000 | 4.5 | 0.013 |
| KRT6A | 6226 | 27650 | 166-500000 | 99574 | 26200 | 13-576000 | 16.0 | 0.780 |
| PDCD1LG2 | 38805 | 16200 | 1-679000 | 98840 | 22600 | 1900-477000 | 2.5 | 0.207 |
| TC2526896 | 4294 | 3185 | 73-27300 | 4571 | 2810 | 274-28600 | 1.1 | 0.609 |
| TBP | 189411 | 167000 | 6000-596000 | 236691 | 142000 | 9730-950000 | 1.2 | |

| | | | | | | | | |
|---|---|---|---|---|---|---|---|---|
| MW¹: Mann-Whitney test | | | | | | | | |

In **Table 4** the expression data of the 17 selected biomarkers in tissue are shown for the groups NBl and BCa total (NMIBC+MIBC). The difference (Fold-Change) between the groups and P-value provide information about/determine the diagnostic performance of the markers. In **Table 5** the data in tissue are shown for the groups NMIBC and MIBC and thereby provide information about the prognostic performance of the biomarkers. In **Tables 6 and 7** the data in the urine samples are shown.

### Summary results examples 1, 2 and 3

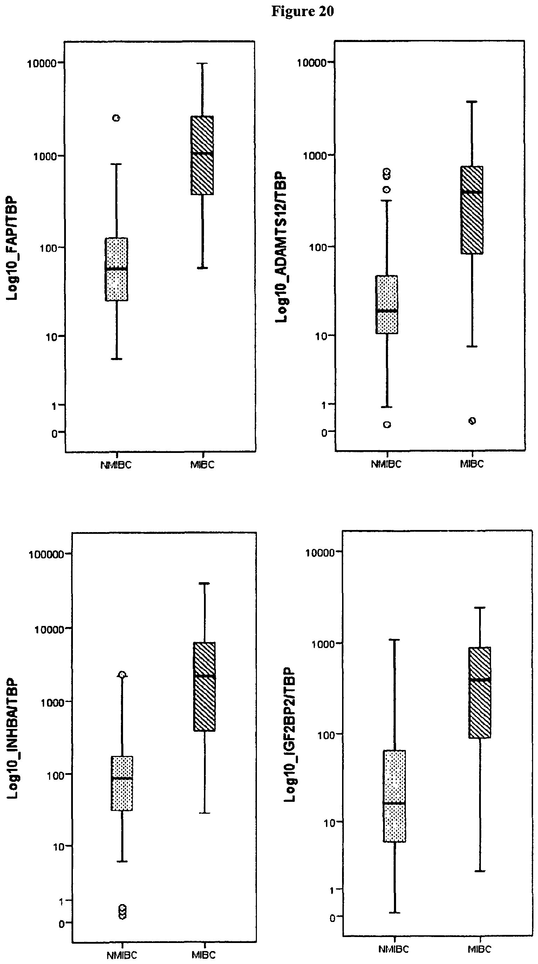

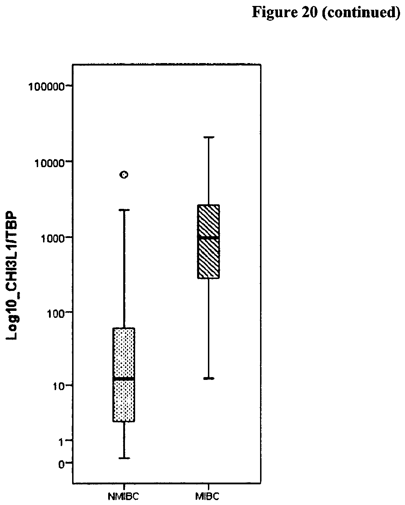

INHBA (**Figures 1****,** **20****; Tables 1, 4-7**): The present GeneChip® Human Exon 1.0 ST Array data, TLDA validation and qPCR assay data showed that INHBA was highly and significantly up-regulated in tissue from MIBC and BC-meta compared to NMIBC tissue. INHBA could also be detected in urine and was highly and significantly up-regulated in urine from BCa patients vs. normal urine. Therefore, INHBA has prognostic value in tissue of patients with BCa and diagnostic value in the detection of BCa in urine.

CTHRC1 (**Figure 2****, Tables 1, 4-7**): The present GeneChip® Human Exon 1.0 ST Array data, TLDA validation and qPCR assay data showed that CTHRC1 was highly and significantly up-regulated in tissue from MIBC and BC-meta compared to NMIBC. CTHRC1 could also be detected in urine and was significantly and highly up-regulated in urine from BCa patients vs. normal urine. Therefore, CTHRC1 has prognostic value in tissue of patients with BCa and diagnostic value in the detection of BCa in urine.

CHI3L1 (**Figures 3****,** **20****: Tables 1, 4-7**): The present GeneChip® Human Exon 1.0 ST Array data, TLDA validation and qPCR assay data showed that CHI3L1 was highly and significantly up-regulated in tissue from MIBC compared to NMIBC. CHI3L1 could also be detected in urine and was significantly and highly up-regulated in urine from BCa patients vs. normal urine. Therefore, CHI3L1 has prognostic value in tissue of patients with BCa and diagnostic value in the detection of BCa in urine.

COL10A1 (**Figures 4****, Tables 1, 4-7**): The present GeneChip® Human Exon 1.0 ST Array data, TLDA validation and qPCR assay data showed that COL10A1 was highly and significantly up-regulated in MIBC and BC-meta compared to NMIBC. COL10A1 could also be detected in urine and was significantly up-regulated in urine from BCa patients vs. normal urine. Therefore, COL10A1 has prognostic value in tissue of patients with BCa and diagnostic value in the detection of BCa in urine.

FAP (**Figures 5****,** **20****,** **21****; Tables 1, 4-7**): The present GeneChip® Human Exon 1.0 ST Array data, TLDA validation and qPCR assay data showed that FAP was highly and significantly up-regulated in tissue from MIBC and BC-meta compared to NMIBC. FAP could also be detected in urine and was significantly and highly up-regulated in urine from BCa patients vs. normal urine and significantly up-regulated in urine from MIBC patients vs. NMIBC patients. Therefore, FAP has prognostic value in urine and in tissue of patients with BCa and diagnostic value in the detection of BCa in urine.

Transcript cluster 2526896 (**Figures 6****, Tables 1, 4-7**): The present GeneChip® Human Exon 1.0 ST Array data, and qPCR assay data showed that transcript cluster TC2526896 was highly and significantly up-regulated in tissue from MIBC and BC-meta compared to NMIBC. Therefore, transcript cluster 2526896 has prognostic value in tissue of patients with BCa.

ASPN (**Figure 7****, Tables 1, 4-7**): The present GeneChip® Human Exon 1.0 ST Array data, TLDA validation and qPCR assay data showed that ASPN was highly and significantly up-regulated in tissue from MIBC compared to NMIBC. Therefore, ASPN has prognostic value in tissue of patients with BCa.

Transcript cluster 2526893 (**Figure 8**): The present GeneChip® Human Exon 1.0 ST Array data showed that transcript cluster 2526893 was highly up-regulated in tissue from MIBC and BC-meta compared to NMIBC. Therefore, transcript cluster 2526893 has prognostic value in tissue of patients with BCa.

ADAMTS 12 (**Figures 9****,** **20****; Tables 1, 4-7**): The present GeneChip® Human Exon 1.0 ST Array data, TLDA validation and qPCR assay data showed that ADAMTS12 was highly and significantly up-regulated in tissue from MIBC and BC-meta compared to NMIBC. Low copy numbers of ADAMTS12 could also be detected in urine. ADAMTS12 was significantly up-regulated in urine from BCa patients vs. normal urine and significantly up-regulated in urine from MIBC patients vs. NMIBC patients. Therefore, ADAMTS12 has prognostic value in urine and tissue of patients with BCa and diagnostic value in the detection of BCa in urine.

IGF2BP2 (**Figures 10****,** **20****; Tables 1, 4-7**): The present GeneChip® Human Exon 1.0 ST Array data, TLDA validation and qPCR assay data showed that IGF2BP2 was highly and significantly up-regulated in tissue from MIBC compared to NMIBC. IGF2BP2 could also be detected in urine and was significantly and highly up-regulated in urine from BCa patients vs. normal urine. Therefore, IGF2BP2 has prognostic value in tissue of patients with BCa and diagnostic value in the detection of BCa in urine.

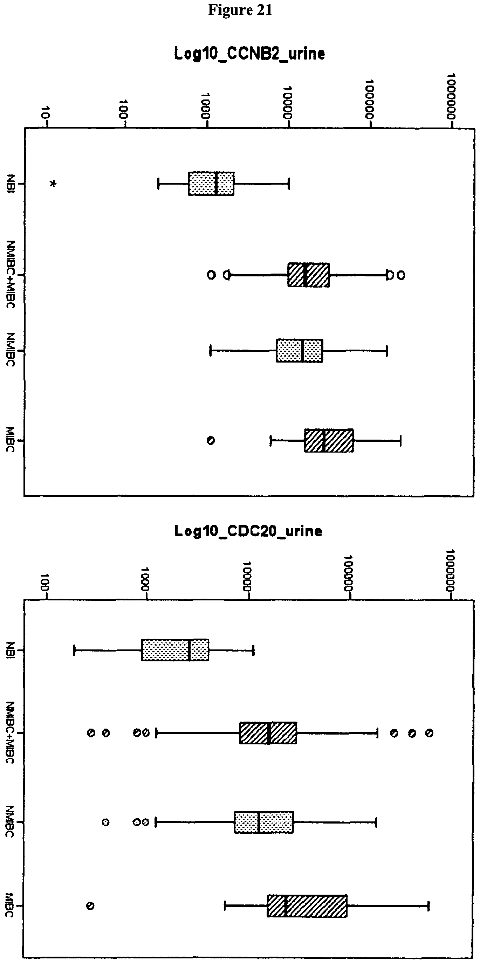

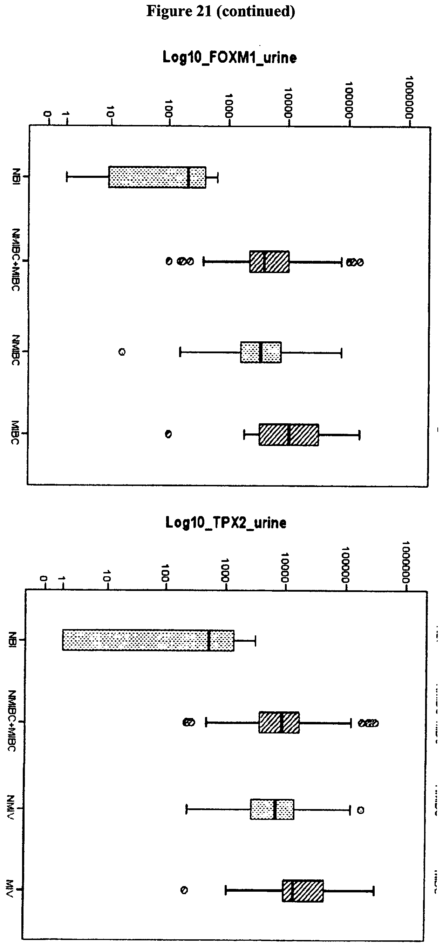

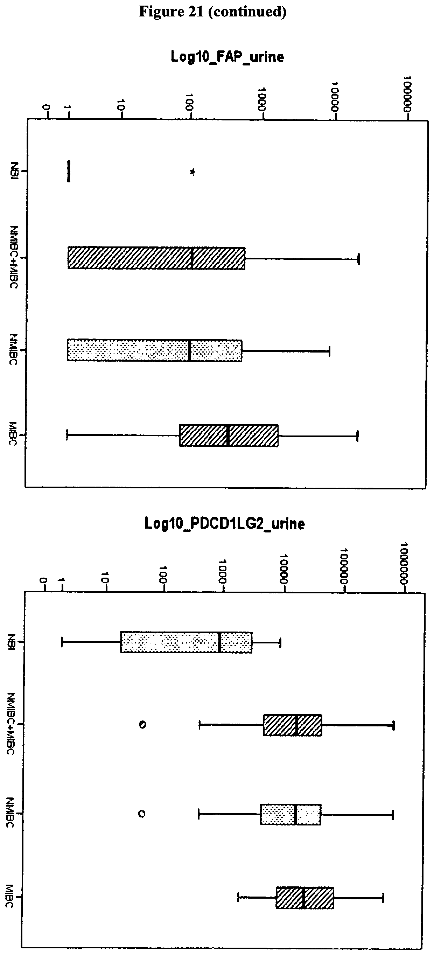

PDCD1LG2 (**Figures 11****,** **21****; Tables 1, 4-7**): The present GeneChip® Human Exon 1.0 ST Array data, TLDA validation and qPCR assay data showed that PDCD1LG2 was significantly up-regulated in tissue from MIBC and BC-meta compared to NMIBC. PDCD1LG2 could also be detected in urine and was significantly and highly up-regulated in urine from BCa patients vs. normal urine. Therefore, PDCD1LG2 has prognostic value in tissue of patients with BCa and diagnostic value in the detection of BCa in urine.

SFRP4 (**Figure 12****; Tables 1, 4-7**): The present GeneChip® Human Exon 1.0 ST Array data, TLDA validation and qPCR assay data showed that SFRP4 was highly and significantly up-regulated in tissue from MIBC and BC-meta compared to NMIBC. Low copy numbers of SFRP4 could also be detected in urine. SFRP4 was significantly up-regulated in urine from BCa patients vs. normal urine. Therefore, SFRP4 has prognostic value in tissue of patients with BCa and diagnostic value in the detection of BCa in urine.

KRT6A (**Figure 13****; Tables 1, 4-7**): The present GeneChip® Human Exon 1.0 ST Array data, TLDA validation and qPCR assay data showed that KRT6A was highly and significantly up-regulated in tissue from MIBC compared to NMIBC. Therefore, KRT6A has prognostic value in tissue of patients with BCa.

TPX2 (**Figures 14****,** **19****,** **21****; Tables 1, 4-7**): The present GeneChip® Human Exon 1.0 ST Array data, TLDA validation and qPCR assay data showed that TPX2 was highly and significantly up-regulated in tissue as well as in urine from patients with BCa compared to normal bladder and significantly up-regulated in tissue and urine from MIBC and BC-meta patients compared to NMIBC patients. Therefore, TPX2 has diagnostic value in tissue and in the detection of BCa in urine and has prognostic value in urine and in tissue of patients with BCa.

CCNB2 (**Figures 15****,** **19****,** **21****; Tables 1, 4-7**): The present GeneChip® Human Exon 1.0 ST Array data, TLDA validation and qPCR assay data showed that CCNB2 was highly and significantly up-regulated in tissue as well as in urine from patients with BCa compared to NB1 and significantly up-regulated in tissue and urine from MIBC and BC-meta patients compared to NMIBC patients. Therefore, CCNB2 has diagnostic value in tissue and in the detection of BCa in urine and has prognostic value in urine and in tissue of patients with BCa.

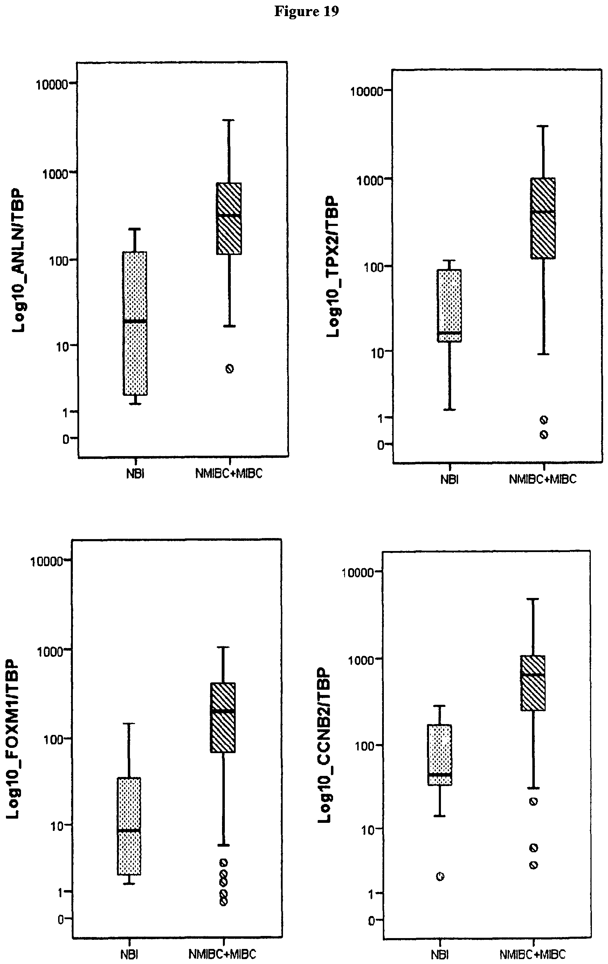

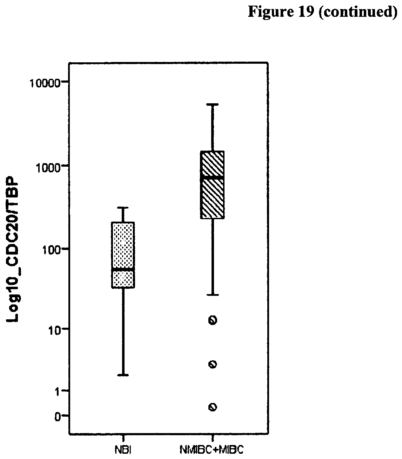

ANLN (**Figures 16****,** **19****; Tables 1, 4-7**): The present GeneChip® Human Exon 1.0 ST Array data, TLDA validation and qPCR assay data showed that ANLN was highly and significantly up-regulated in tissue as well as in urine from patients with BCa compared to NBl and significantly up-regulated in tissue from MIBC and BC-meta patients compared to NMIBC patients. Therefore, ANLN has diagnostic value in tissue and in the detection of BCa in urine and has prognostic value in tissue of patients with BCa.

FOXM1 (**Figures 17****,****19****,** **21****; Tables 1, 4-7**)**:** The present GeneChip® Human Exon 1.0 ST Array data, TLDA validation and qPCR assay data showed that FOXM1 was highly and significantly up-regulated in tissue as well as in urine from patients with BCa compared to NBl and significantly up-regulated in tissue and urine from MIBC and BC-meta patients compared to NMIBC patients. Therefore, FOXM1 has diagnostic value in tissue and in the detection of BCa in urine and has prognostic value in urine and in tissue of patients with BCa.

CDC20 (**Figures 18****,** **19****,** **21****; Tables 1, 4-7**): The present GeneChip® Human Exon 1.0 ST Array data, TLDA validation and qPCR assay data showed that CDC20 was highly and significantly up-regulated in tissue as well as in urine from patients with BCa compared to NBl and significantly up-regulated in tissue and urine from MIBC and BC-meta patients compared to NMIBC patients. Therefore, CDC20 has diagnostic value in tissue and in the detection of BCa in urine and has prognostic value in urine and in tissue of patients with BCa.

### Example 4: Selection of the best candidate biomarkers

Based on the highest up-regulation in BCa vs NBl and MIBC vs. NMIBC, lowest P-value and high copy numbers the best performing diagnostic and prognostic individual biomarkers in tissue and urine were identified. The five best performing individual biomarkers for the detection of BCa in tissue were identified and are shown in boxplots in **Figure 19**: ANLN, TPX2, FOXM1, CCNB2 and CDC20.

The five best performing individual biomarkers that could distinguish MIBC tissue from NMIBC tissue were identified and are shown in boxplots **in** **Figure 20**: IGF2BP2, INHBA, ADAMTS12 FAP and CHI3L1.

The six best performing individual biomarkers for the detection of BCa in urine were identified and are shown in a boxplot in **Figure 21**: FAP, TPX2, CCNB2, CDC20, FOXM1 and PDCD1LG2. The first five genes could also significantly distinguish MIBC from NMIBC in urine.

Given that the nature of these tumors is very heterogeneous, it is likely that combination of markers can identify different patients and have additional diagnostic and/or prognostic value to eachother. For the identification of the best combinations of biomarkers for the diagnosis of BCa in urine and/or tissue and for the best combinations of markers that had prognostic value by distinguishing MIBC from NMIBC in tissue and/or urine the method of binary logistic regression analysis was performed. All data were log-transformed prior to statistical analysis as a transformation to a normal distribution. Binary logistic regression analysis (stepwise forward) was performed with the 17 biomarkers in order to find regression models and marker combinations for predicting the presence of bladder cancer (NMIBC and MIBC) in urine or for predicting whether BCa is muscle invasive or not. The statistical significant level for all tests was set at P=0.05.

As example two possible identified combinations of biomarkers are described, one for predicting the occurrence of BCa based on the expression of the markers in urine and one for predicting the occurence of muscle invasive disease based on the expression of the markers in tissue.

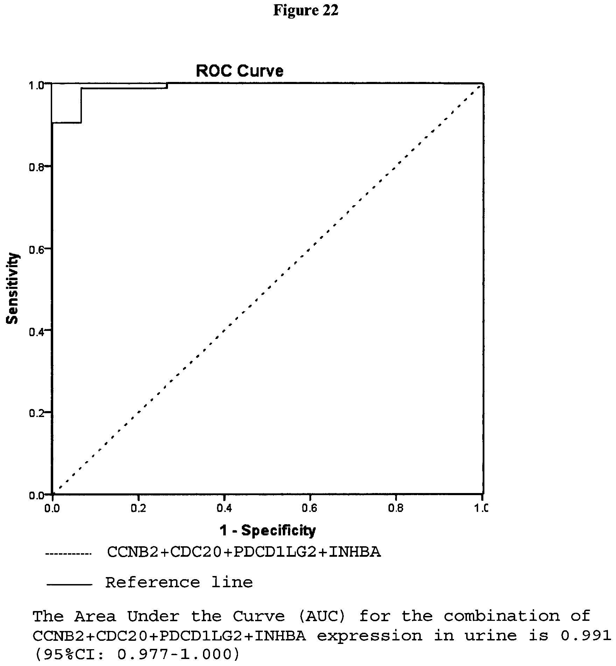

In urine, CCNB2 is a key predictor and predicts that 66.7% of healthy controls have no cancer and that 96.5% from the cancer patients do have cancer. With the addition of CDC20 and PDCD1LG2 the new model predicts that 80% of the healthy controls have no cancer and 96.5% of the cancer patients are correctly classified. When INHBA is added to this model the model model predicts that 93.3% of the healthy controls have no cancer and that 98.8% of the cancer patients are correctly calssified. This four biomarker model is higly significant (P=1.9E-13) showing that the biomarkers can predict the presence of bladdercancer in urine well. To visualize the performance of the biomarker combinations a ROC curve is shown (**Figure 22**).

In a ROC curve, the true positive rate to detect BCa or MIBC (sensitivity) is plotted in function of the false positive rate (i.e. positives in the control group, 1-specificity) for different cut-off points. Each point on the curve represents a sensitivity/specificity pair corresponding to a particular decision threshold. The Area Under the Curve (AUC) of the ROC curve is a measure how well a parameter can distinguish between two groups and is maximum 1.0 (all samples correctly classified). The AUC for the combination of CCNB2, CDC20, PDCD1LG2 and INHBA expression is 0.991 (95%CI: 0.977-1.000).

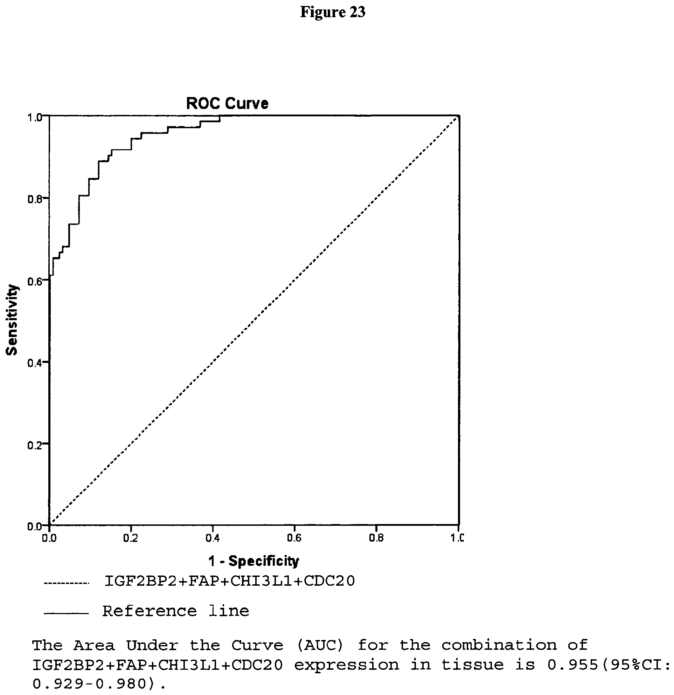

In tissue, FAP is a key predictor and predicts that 87% of the NMIBC are NMIBC and that 80.3% of the MIBC specimen are correctly classified. When CDC20 and CHI3L1 are added 89.3 of the NMIBC are correctly classified and 83.1% of the MIBC are correctly classified. The addition of IGF2BP2 leads to the correct classification of 90.2% of the NMIBC and 83.1% of the MIBC. This four marker model is higly significant (P=4.9 x10-32) showing that the biomarkers can predict the occurence of muscle invasive disease well. The Area Under the Curve (AUC) for the combination of IGF₂BP₂+FAP+CHI₃L₁+CDC₂₀ expression is 0.955 (95%CI: 0.929-0.980). See **Figure 23**.

Based on the binary logistic regression model the following genes and combinations were identified. For predicting the occurrence of BCa (diagnosis) based on the detection and quantification expression of the markers in tissue: at least ANLN combined with one or more markers from the list: IGF2BP2, FAP, CTHRC1, CCNB2, COL10A1 and/or TPX2. For predicting the occurence of muscle invasive disease (prognosis) based on the expression of the markers in tissue: at least FAP, combined with one or more markers from the list: CDC20, CHI3L1, IGF2BP2, INHBA, ADAMTS12, CCNB2 and/or ANLN or at least CHI3L1, combined with one or more markers from the list: CDC20, FAP, IGF2BP2, INHBA, ADAMTS12, CCNB2 and/or ANLN.

For predicting the occurrence of BCa based on the expression of the markers in urine at least CCNB2, combined with one or more markers from the list: CDC20, PDCD1LG2, TPX2, SFRP4, COL10A1, INHBA and/or TC2526896 or at least PDCD1LG2, combined with one or more markers from the list: CCNB2, CDC20, TPX2, SFRP4, COL10A1, INHBA and/or CTHRC1

For predicting the occurence of muscle invasive disease based on the expression of the markers in urine at least FAP, combined with one or more from the list FOXM1, CCNB2, CDC20 and/or TC2526896

### CONCLUSIONS:

The present invention relates to biomarkers and their diagnostic and prognostic uses for bladder cancer. The biomarkers can be used alone or in combination. The invention provides methods for diagnosing bladder cancer in a subject, comprising measuring the levels of a single or a plurality of biomarkers in a biological sample derived from a subject suspected of having bladder cancer. Differential expression of one or more biomarkers in the biological sample is compared to one or more biomarkers in a healthy control sample indicates that a subject has cancer. Furthermore, the invention provides methods for determing classification of tumors according to the aggressiveness or establishing the prediction of prognosis and disease outcome for a human individual suffering from bladder cancer, comprising measuring the levels of a single or a plurality of biomarkers in a biological sample derived from a subject having bladder cancer. Differential expression of one or more biomarkers in the biological sample is compared to one or more biomarkers in a NMIBC control sample that indicates that a subject has an aggressive type of bladder cancer.

Based on the results obtained and described in examples 1, 2, 3 and 4, the following observations can be made:
1) Given that the biological sample is urine, the identified best performing individual biomarkers for diagnosis of BCa were: FAP, TPX2, CCNB2, CDC20, FOXM1 and PDCD1LG2. The first five markers could also significantly distinguish MIBC from NMIBC in urine and therefore had prognostic value.
2) The best combinations of biomarkers for predicting the occurrence of BCa based on the expression of the markers in urine contain at least CCNB2, combined with one or more markers from the list: CDC20, PDCD1LG2, TPX2, SFRP4, COL10A1, INHBA and/or TC2526896; or contain at least: PDCD1LG2, combined with one or more markers from the list: CCNB2, CDC20, TPX2, SFRP4, COL10A1, INHBA and/or CTHRC1;
3) The best combination of biomarkers for predicting the occurrence of muscle invasive disease based on the expression of the markers in urine contains at least FAP, combined with one or more from the list FOXM1, CCNB2, CDC20 and/or TC2526896;
4) Given that the biological sample is tissue, the identified best performing individual biomarkers for diagnosis of BCa were: ANLN, TPX2, FOXM1, CCNB2 and CDC20;
5) The identified best performing individual biomarkers that could distinguish MIBC tissue from NMIBC tissue were: IGF2BP2, INHBA, ADAMTS12 FAP and CHI3L1;
6) The best combination of biomarker s for predicting the occurrence of BCa based on the expression of the markers in tissue contains at least ANLN combined with one or more markers from the list: IGF2BP2, FAP, CTHRC1, CCNB2, COL10A1 and/or TPX2;
7) The best combinations of biomarkers for predicting the occurrence of muscle invasive disease based on the expression of the markers in tissue contain at least FAP, combined with one or more markers from the list: CDC20, CHI3L1, IGF2BP2, INHBA, ADAMTS12, CCNB2 and/or ANLN or at least CHI3L1, combined with one or more markers from the list: CDC20, FAP, IGF2BP2, INHBA, ADAMTS12, CCNB2 and/or ANLN.

## Claims

1. Method for establishing the prediction of prognosis and disease outcome for a human individual suffering from bladder cancer comprising:
a) determining the expression of CCNB2 in a sample originating from said human individual; and
b) establishing up regulation of expression of CCNB2 as compared to expression of CCNB2 in a sample originating from said human individual not comprising tumour cells or tissue, or from an individual, or group of individuals, not suffering from bladder cancer; and
c) establishing the prediction of prognosis and disease outcome for a human individual suffering from bladder cancer based on the established upregulation of CCNB2
wherein expression analysis is performed on a sample selected from the group consisting of urine and urine sediment.

2. Method according to claim 1, wherein determining the expression comprises determining mRNA expression of the one or more genes, preferably by Northern blot hybridisation or amplification based techniques, preferably PCR, real time PCR, or NASBA.

3. Method according to claim 1 or claim 2, wherein expression analysis comprises high-throughput array chip analysis.

## Patentansprüche

1. Verfahren zur Erzeugung der Vorhersage einer Prognose und eines Krankheitsausgangs für ein menschliches Individuum, das unter Blasenkrebs leidet, das aufweist:
a) Bestimmen der Expression von CCNB2 in einer Probe, die von diesem menschlichen Individuum stammt; und
b) Erzeugen einer Heraufregulierung der Expression von CCNB2 im Vergleich zu der Expression von CCNB2 in einer Probe, die von dem menschlichen Individuum, das keine Tumorzellen oder Gewebe aufweist, oder von einem Individuum oder einer Gruppe von Individuen, die nicht unter Blasenkrebs leiden, stammt; und
c) Erzeugen der Vorhersage einer Prognose und eines Krankheitsausgangs für ein menschliches Individuum, das unter Blasenkrebs leidet, basierend auf der erzeugten Aufwärtsregulierung von CCNB2,
wobei eine Expressionsanalyse an einer Probe ausgeführt wird, die aus der Gruppe ausgewählt wird, die aus Urin und Urinsediment besteht.

2. Verfahren nach Anspruch 1, wobei die Bestimmung der Expression die Bestimmung der mRNA-Expression des einen oder mehrerer Gene, vorzugsweise durch Techniken auf Basis von Northern-Blot-Hybridisierung oder Vervielfältigung, vorzugsweise PCR, Echtzeit-PCR oder NASBA, aufweist.

3. Verfahren nach Anspruch 1 oder 2, wobei die Expressionsanalyse eine Hochdurchsatzanaordnungschipanalyse aufweist.

## Revendications

1. Procédé pour établir la prédiction du pronostic et de l'issue de la maladie pour un individu humain souffrant d'un cancer de la vessie comprenant :
a) la détermination de l'expression de CCNB2 dans un échantillon provenant dudit individu humain ; et
b) l'établissement de la régulation positive de l'expression de CCNB2 comparativement à l'expression de CCNB2 dans un échantillon provenant dudit individu humain ne comprenant pas des cellules ou du tissu tumoral ou provenant d'un individu, ou d'un groupe d'individus, ne souffrant pas d'un cancer de la vessie ; et
c) l'établissement de la prédiction du pronostic et de l'issue de la maladie pour un individu humain souffrant d'un cancer de la vessie sur la base de la régulation positive établie de CCNB2
dans lequel l'analyse de l'expression est effectuée sur un échantillon choisi dans le groupe constitué de l'urine et du sédiment urinaire.

2. Procédé selon la revendication 1, dans lequel la détermination de l'expression comprend la détermination de l'expression de l'ARNm des un ou plusieurs gènes, de préférence par une hybridation Northern blot ou des techniques fondées sur l'amplification, de préférence la PCR, la PCR en temps réel, ou la technique NASBA.

3. Procédé selon la revendication 1 ou la revendication 2, dans lequel l'analyse de l'expression comprend l'analyse de puces de réseau haut-débit.
